# EUROPEAN PATENT APPLICATION

(11) **EP 1 541 145 A1**
(43) Date of publication of application: **15.06.2005**
(21) Application number: 03739961.5
(22) Date of filing: 19.06.2003
(51) Int. Cl.: A61K 31/41, A61P 9/10

(54) **THE USE OF BENZISOSELENAZOLONE COMPOUNDS AGAINST ISCHEMIC MYOCARDIAL DAMAGE**

(30) Priority: 19.06.2002 CN 02122664
(71) Applicant: Institute of Materia, Chinese Academy of Medical Sciences, Xuanwu District 100050 (CN)
(72) Inventor: WANG, Xiaoliang, Xuanwu District, 100050 (CN); GOU, Zongru, Xuanwu District, 100050 (CN); LU, Jing, Xuanwu District, 100050 (CN); CHU, Fengming, Xuanwu District, 100050 (CN); PAN, Yaping, Xuanwu District, 100050 (CN); WANG, Ling, Xuanwu District, 100050 (CN)
(74) Representative: Daniels, Jeffrey Nicholas
(86) International application number: PCT/CN2003/000475
(87) International publication number: WO 2004/000309

(57) **Abstract**

The invention discloses use of benzisoselenazolones, particularly compounds represented by the general formula (I) against ischemic myocardial injury. The compounds are **characterized by** selectively inhibiting Na⁺/Ca²⁺ exchange, dilating coronary artery and decreasing myocardial oxygen consumption, which possess the advantages of high activity, potent specificity and low toxicity.

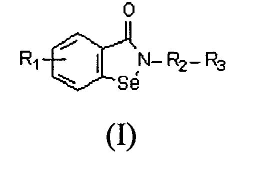

## Description

### Technical Field

The present invention relates to the use of benzisoselenazolones, especially the use thereof as drugs against ischemic myocardial injury.

### Background Art

With the increase of living condition and the progress of aging, the invasion of cardiovascular diseases, especially ischemic myocardial injury, are increasing and have been an important factor affecting quality of life and life of the elderly people. Currently clinically used drugs, such as nitrate vasodilators, dihydropyridine calcium channel antagonists as well as β-adrenergic blockers, are all restricted due to the side effects.

The Na⁺/Ca²⁺ exchanger is an important ion-exchange system on the excitatory cell membrane, ofwhich the principle function is to extrude the intracellular calcium over the Na⁺/Ca²⁺ exchange to restore the intracellular calcium to an inactivate level. During the Na⁺/Ca²⁺ exchange, three (3) Na⁺ are transported into the cell per extrusion of one (1) Ca²⁺, thus there is a net charge cross-membrane movement which induces a cross-membrane current. Na⁺-Ca²⁺ exchange is a biphasical process, at the early stage of myocardial ischemia/reperfusion, myocardial cell membrane injury results in the abnormality of membrane potential and alteration of membrane permeability, large amount of Na⁺ enter into the myocardial cell, and the Na⁺/Ca²⁺ exchanger is reverse-activated to extrude excessive Na⁺ and results in intracellular Ca²⁺ overload, which exacerbates injury and death of the myocardial cells. Such reverse transportations, under pathological state, may result in severe after-effects. Thus, the Na⁺/Ca²⁺ reverse-exchanger is deemed to be an important mechanism of the adverse effects of ischemia/reperfusion. Up to now, there is no drug that can specifically block the Na⁺/Ca²⁺ reverse-exchange all over the world.

US patent No. 4, 711, 961 has disclosed a preparation method of benzisoselenazolones and predicted use of the compounds for the prophylaxis and therapy of infectious diseases to stimulate the immune system and for the therapy of selenium deficiency diseases, arteriosclerosis, rheumatic diseases (especially arthrosis), but no experimental or use evidences are provided. Benzisoselenazolones are a series of synthetic compounds, which mimic the activity of glutathione peroxide reductase. Among the compounds, Ebselen, a representative drug, shows anti-inflammatory effect and is clinically used for the treatment of subarachnoid hemorrhage. However, no report about the inhibitory effect on the Na⁺/Ca²⁺ exchanger as well as the protective effect thereof on myocardial ischemia has been reported yet.

### Disclosure of the Invention

An object of the present invention is to provide use of the benzisoselenazolones or a composition containing the same for the prevention and/or treatment of ischemic myocardial injury.

One other aspect of the invention is to provide a pharmaceutical composition which comprises the benzisoselenazolone compounds as an active ingredient and an ordinary carrier in pharmaceutical field.

Another aspect of the invention is to provide use of a benzisoselenazolone compound or a pharmaceutical composition thereof as a drug against ischemic myocardial injury.

Yet one another aspect of the invention is to provide a prevention and/or treatment method of ischemic myocardial injury, comprising administering to the being suffering from such states the benzisoselenazolone compounds or a pharmaceutical composition containing the same.

The Na⁺/Ca²⁺ exchanger is an important biphasical ion transport protein locating on the excitatory cell membrane, ofwhich the principle function is to extrude the Ca²⁺ ion entered during the exciting stage and restore the intracellular calcium to an inactivate level. During the Na⁺/Ca²⁺ exchange, three Na⁺ are transported into the cell per extrusion of each Ca²⁺, thus there is a net charge cross-membrane movement which induces a cross-membrane current, which is an inward current. At the early stage of myocardial ischemia/reperfusion, myocardial cell membrane injury results in the abnormality of membrane potential and an alteration of membrane permeability, large amounts of Na⁺ enters into the myocardial cell, and the Na⁺/Ca²⁺ exchanger is reverse-activated to extrude excessive Na⁺ and results in intracellular Ca²⁺ overload, which exacerbates injury and death of the myocardial cells. Hence, the Na⁺/Ca²⁺ reverse-exchanger is deemed to be an important mechanism of the adverse effects of ischemia/reperfusion.

Being a cross-membrane protein per se, the Na⁺/Ca²⁺ exchanger is an important target for drug actions. Up to now, there is no drug reported which directs to the protein and shows protective effect on myocardial cells. Therefore, it is of great benefit to develop selective blockers of Na⁺/Ca²⁺ exchanger for the treatment of ischemic myocardial injury (coronary heart disease) and protection of heart.

Upon the research of present invention, it illustrates that benzisoselenazolone compounds can significantly block Na⁺/Ca²⁺ exchange. The present compounds are novel compounds against ischemic myocardial injury which effect on the Na⁺/Ca²⁺ exchange system.

Cell level experiments illustrate that, comparing with Amiloride and KB-R7943, the present compounds are more specific and selective on the target protein, Na⁺/Ca²⁺ exchanger. Comparative study of Amiloride, KB-R7943 and the present compounds on the effects to the Na⁺/Ca²⁺ exchange current were conducted and the results demonstrated that Amiloride in low concentration has little effect on inward and outward current, while high level Amiloride represents inhibitory effect on outward I_{Na-Ca}; KB-R7943 has inhibitory effect on biphasical Na⁺/Ca²⁺ exchange with no selectivity, the inward and outward I_{Na-Ca} decreased simultaneously, which suggests the inhibitory effect on normal function of membrane to extrude Ca²⁺; the present compounds show more selectively and potent inhibitory effect on outward I_{Na-Ca} than inward I_{Na-C}, which is beneficial to inhibit the Ca²⁺ overload caused by ischemia/reperfusion process and the resulted myocardial cell injury. The present compounds are all inhibitory on reverse mode of Na⁺/Ca²⁺ exchange process, ofwhich the potent ones have an inhibitory ratio of higher than 40%, and that of the most potent ones is nearly 60%.

The inhibitory effects to the reverse mode of Na⁺/Ca²⁺ exchange process of the present compounds are illustrated via a cell level pathological model, moreover, the effects against myocardial ischemic injury of the compound are also confirmed via *in vitro* and *in vivo* tests.

Based on the pre-electrophysiological cell model screening, we have compared the inhibition ratios of the outward current by reverse-transportation of Na⁺/Ca²⁺ exchange, and further *in vitro* tests on isolated rat hearts subjected to ischemia-reperfusion (I/R) were conducted. Prolongation of ventricular arrhythmic, incidence rate of ventricular extrasystole, ventricular tachycardia/ventricular fibrillation (VT/VF) were recorded to evaluate the protective actions of drugs on myocardial injury. The result demonstrates the significant protective effect of the present compounds on cardiac cell injury.

Protective effect of the present compounds in rats after intravenous administration: the present compounds can significantly prolong the latency of ischemia-reperfusion ventricular arrhythmic, decrease the incidence and duration of VT and VF. Such results indicate that the tested compounds have protective effects against coronary arterial ischemia/ reperfusion-induced myocardial injury in rats. Antiarrhythmic potency is used to assess the protection of tested compounds on myocardial injury. The same result was also observed in rats orally treated with tested compounds. Rats orally administered with tested compounds, and the left anterior descending coronary artery was ligated 60 minutes after the administration, and reperfusion was performed 15 minutes after the ligation. The present compounds can significantly decrease the duration of ischemia-reperfusion ventricular arrhythmic (P<0.01), decrease the incidence and duration of VT (P<0.01) and VF (P<0.05). This indicates that the present compounds have protective effects against coronary arterial ischemia/reperfusion-induced injury.

The present compounds can significantly improve acute myocardial ischemia and myocardial infarction in dogs, the severity (Σ-ST) and area (NST) of myocardial ischemia revealed by epicardial electrography, and the infarction size represented by N-BT staining method can all be reduced. The present compounds can evidently reduce the severity (Σ-ST) myocardial ischemia, Σ-ST reduced by 53.90±20.60% 180 minutes after the administration of the tested compounds, with significant difference to controls and Σ-ST observed before administration (P<0.01). The results revealed by epicardial electrography showed that the present compounds can evidently improve the dogs subjected to experimental acute myocardial ischemia, the severity (Σ-ST) and area (NST) of myocardial ishemia were significantly reduced. The present compound can reduce the myocardial infarction size. Myocardial infarct size represented by quantitative histological N-BT staining method was reduced by 69.18% and 67.94% after administration of the present compounds, with significant difference to the control group (P<0.001). The present compounds significantly inhibit elevation of the activities of CK and AST, while have little effect on the activity of LDH comparing with the controls.

The present compounds demonstrate effects on coronary arterial vasodilatation, can significantly increase the coronary flow in the ischemia and myocardial infarct, promote the coronary artery vasodilatation as well as the collateral circulation, and ameliorate blood supply in the ischemic region. Coronary flow in the ischemia and myocardial infarct was significantly increased by 15.02±22.16% verse the baseline 30 minutes after administration, with significant difference to the control group (P<0.05). The ventricular oxygen content was significantly increased after administration of the present compounds, with significant difference verse prior administration (P<0.05). The results obtained from the experiment of dogs subjected to acute myocardial ischemia and infarction showed that the pathological changes were inhibited by the present compounds, the severity of myocardial ischemia and infarction as well as the infarction size were reduced; meanwhile coronary vasodilatation was induced as well as coronary flow was increased in the administration group, further, the elevation of CK activity and the release of AST were all inhibited. These results consist with the mechanism of the actions, that is, selectively inhibiting the reverse mode of Na⁺/Ca²⁺ exchange process and the related myocardial ischemia damage, inducing coronary artery vasodilatation, and ameliorating cardiac blood supply.

The present compounds have little effect on cardiac function and hemodynamics in normal rats. The results obtained from anesthetic normal rat pretreated with the present compounds revealed that the present compounds have little affect on left ventricular systolic pressure (LVSP), left ventricular end diastolic pressure (LVEDP), maximum rate of left ventricular pressure (±dp/dtmax), arterial systolic pressure (SP), arterial diastolic pressure (DP), mean arterial pressure (MAP), and heart rate in rats. The action of the present compounds on above study items has no significant difference and no statistical difference comparing with the vehicle controls.

The present compounds can ameliorate the cardiac function and hemodynamics in rats subjected to myocardial ischemia. LVSPs of rats subjected to myocardial ischemia/reperfusion were well preserved in the compounds administered groups, while the fluctuation of LVSPs were greater in the model control group. Reperfusion triggered a rapid increase in LVEDP after 10 min reperfusion, indicating dilative function of left ventricle was impaired and damaged seriously; while the LVEDPs of rats administered with the present compounds were well preserved to the level of baseline, indicating the potent protective effect on cardiac diastolic function in rats subjected to myocardial ischemia. No significant effect of the present compounds on +dp/dtmax was observed. +dp/dtmax in model group decreased more than the compound administration group, the value of +dp/dtmax in the model control group was 54% lower than the compound administration group. This result suggests that the impairment of contractile function during ischemia/perfusion injury was ameliorated by the present compounds' treatment. -dp/dtmax is a parameter indicating the function of myocardial dilastolization, which is one of the most important items to assess ventricular diastolic function. There are significant difference between the compounds treatment group and vehicle control group at each time-point after ligation. Myocardial contractile function is reduced by the present compounds, while the diastolic function is well preserved. Blood pressure stands stably after the administration of the present compounds owing to the protective effect on cardiac function. Blood pressure of the model control group reduces in a large-scale and fluctuates greater due to severe injury. Heart rate decreases in rats subjected to ischemia/reperfusion injury and the present compounds have little effect on heart rate and there is no difference between the two groups. Cardiac function is impaired by ischemia/reperfusion insult, representing by the decrease of cardiac contractile and diastolic function as well as blood pressure decrease or fluctuate greatly. The present compounds show protective effect on cardiac dysfunction in rats, with more significant effect on the improvement of cardiac diastolic function. Basically, the cardiac diastolic function in compound administration group is preserved to normal level. Blood pressure is stable in compound treatment group too. And the present compounds have little effect on the decrease of heart rate induced by myocardial insult.

Toxicology study indicates that the present compounds have no mutagenicity and high safety as well as low toxicity. The results were all negative in Ames (mutagenesis) tests performed with different concentration of compounds and diverse bacteria strains. In acute toxicity studies, there was no mortality in any of the doses, LD₅₀ (50% lethal dose) >5 g/kg.

The benzisoselenazolone compounds of the present invention, include but not limited to, compounds represented by formula (I), 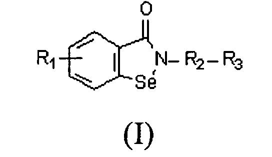 wherein R₁ is selected from a hydrogen, a halogen, a C₁-C₄ linear or branched alkyl, a C₁-C₄ linear or branched alkoxy, a hydroxy, a trifluoromethyl, a nitro, a di-(C₁-C₄ alkyl)-amino, or a methylenedioxy group, the substitution site of R₁ may be any one of the 3-, 4-, 5- or 6-position of the benzene ring of benzisoselenolone;
R₂ represents a C₀-C₄ saturated or unsaturated alkyl (R₂ is a bond connecting R3 and nitrogen while R₂ being C₀), preferably R₂ represents a C₀-C₂ saturated or unsaturated alkyl, more preferably a C₀-C₁ saturated or unsaturated alkyl, and more preferably C₀, i.e., R₂ is a single bond connecting R₃ and nitrogen atom.
R3 represents a saturated or unsaturated, substituted or unsubstituted phenyl or heterocyclic radical, said heterocyclic radical preferably having 1 to 4 hetero atoms, more preferably 1 to 2 hetero atoms, said hetero atoms being selected from oxygen, nitrogen or sulphur, more preferably, said R3 being selected from phenyl, phenylthio, thiazolyl, isothiazolyl, imidazolyl, pyrazolyl, thiadiazolyl, pyridyl, pyrimidyl, pyrazinyl, pyridazinyl, benzothiazolyl, benzimidazolyl, benzotriazolyl, triazinyl, triazolyl, tetrazolyl, quinolinyl, isoquinolinyl, indolyl, indazolyl, carbazolyl, furyl;most preferably, R3 is selected from phenyl and pyridyl;

Said heterocyclic radical may be mono- or di- substituted, said substituents may be identical or different, and being selected from the group consisting of halogen, C₁-C₄ linear or branched alkyl, C₁-C₄ linear or branched alkoxy, C₁-C₄ linear or branched alkylthio, C₁-C₄ linear or branched keto, C₁-C₄ linear or branched haloalkyl, hydroxy, thio, nitro, cyano, carboxyl, and alkoxycarbonyl;

The preferred substituent is selected from the group consisting of halogen, C₁-C₄ linear or branched alkyl, C₁-C₄ linear or branched alkoxy, C₁-C₄ linear or branched alkylthio, C₁-C₄ linear or branched haloalkyl, C₁-C₄ linear or branched keto, hydroxy, thio, nitro, cyano, carboxyl and alkoxycarbonyl.

More preferred substituent is selected from fluoro, chloro, bromo, methyl, ethyl, butyl, methoxy, ethoxy, methylthio, ethylthio, trifluoromethyl, hydroxy, thio, nitro, cyano, carboxyl and alkoxycarbonyl.

Most preferred substituent is selected from fluoro, chloro, bromo, methyl, methoxy, ethoxy, methylthio, hydroxy, thio, nitro, carboxyl, methoxycarbonyl and ethoxycarbonyl.

The most preferred compounds according to the invention are selected from following:
2-phenyl-1, 2-benzisoselenazol-3(2H)-one,
4-fluoro-2-phenyl-1, 2-benzisoselenazol-3(2H)-one,
5-fluoro-2-phenyl-1, 2-benzisoselenazol-3(2H)-one,
6-fluoro-2-phenyl-1, 2-benzisoselenazol-3(2H)-one,
7-fluoro-2-phenyl-1, 2-benzisoselenazol-3(2H)-one,
4-chloro-2-phenyl-1, 2-benzisoselenazol-3(2H)-one,
5-chloro-2-phenyl-1, 2-benzisoselenazol-3(2H)-one,
6-chloro-2-phenyl-1, 2-benzisoselenazol-3(2H)-one,
7-chloro-2-phenyl-1, 2-benzisoselenazol-3(2H)-one,
4-hydroxy-2-phenyl-1, 2-benzisoselenazol-3(2H)-one,
5-hydroxy-2-phenyl-1, 2-benzisoselenazol-3(2H)-one,
6-hydroxy-2-phenyl-1, 2-benzisoselenazol-3(2H)-one,
7-hydroxy-2-phenyl-1, 2-benzisoselenazol-3(2H)-one,
2-(2-pyridyl)-1, 2-benzisoselenazol-3(2H)-one,
2-(3-pyridyl)-1, 2-benzisoselenazol-3(2H)-one,
2-(4-pyridyl)-1, 2-benzisoselenazol-3(2H)-one,
4-chloro-2-(2-pyridyl)-1, 2-benzisoselenazol-3(2H)-one,
4-chloro-2-(3-pyridyl)-1, 2-benzisoselenazol-3(2H)-one,
4-chloro-2-(4-pyridyl)-1, 2-benzisoselenazol-3(2H)-one,
5-chloro-2-(2-pyridyl)-1, 2-benzisoselenazol-3(2H)-one,
5-chloro-2-(3-pyridyl)-1, 2-benzisoselenazol-3(2H)-one,
5-chloro-2-(4-pyridyl)-1, 2-benzisoselenazol-3(2H)-one,
6-chloro-2-(2-pyridyl)-1, 2-benzisoselenazol-3(2H)-one,
6-chloro-2-(3-pyridyl)-1, 2-benzisoselenazol-3(2H)-one,
6-chloro-2-(4-pyridyl)-1, 2-benzisoselenazol-3(2H)-one,
7-chloro-2-(2-pyridyl)-1, 2-benzisoselenazol-3(2H)-one,
7-chloro-2-(3-pyridyl)-1, 2-benzisoselenazol-3(2H)-one,
7-chloro-2-(4-pyridyl)-1, 2-benzisoselenazol-3(2H)-one,
4-fluoro-2-(2-pyridyl)-1, 2-benzisoselenazol-3(2H)-one,
4-fluoro-2-(3-pyridyl)-1, 2-benzisoselenazol-3(2H)-one,
4-fluoro-2-(4-pyridyl)-1, 2-benzisoselenazol-3(2H)-one,
5-fluoro-2-(2-pyridyl)-1, 2-benzisoselenazol-3(2H)-one,
5-fluoro-2-(3-pyridyl)-1, 2-benzisoselenazol-3(2H)-one,
5-fluoro-2-(4-pyridyl)-1, 2-benzisoselenazol-3(2H)-one,
6-fluoro-2-(2-pyridyl)-1, 2-benzisoselenazol-3(2H)-one,
6-fluoro-2-(3-pyridyl)-1, 2-benzisoselenazol-3(2H)-one
6-fluoro-2-(4-pyridyl)-1, 2-benzisoselenazol-3(2H)-one,
7-fluoro-2-(2-pyridyl)-1, 2-benzisoselenazol-3(2H)-one,
7-fluoro-2-(3-pyridyl)-1, 2-benzisoselenazol-3(2H)-one,
7-fluoro-2-(4-pyridyl)-1, 2-benzisoselenazol-3(2H)-one,
4-hydroxy-2-(2-pyridyl)-1, 2-benzisoselenazol-3(2H)-one,
4-hydroxy-2-(3-pyridyl)-1, 2-benzisoselenazol-3(2H)-one,
4-hydroxy-2-(4-pyridyl)-1, 2-benzisoselenazol-3(2H)-one,
5-hydroxy-2-(2-pyridyl)-1, 2-benzisoselenazol-3(2H)-one,
5-hydroxy-2-(3-pyridyl)-1, 2-benzisoselenazol-3(2H)-one,
5-hydroxy-2-(4-pyridyl)-1, 2-benzisoselenazol-3(2H)-one,
6-hydroxy-2-(2-pyridyl)-1, 2-benzisoselenazol-3(2H)-one,
6-hydroxy-2-(3-pyridyl)-1, 2-benzisoselenazol-3(2H)-one,
6-hydroxy-2-(4-pyridyl)-1, 2-benzisoselenazol-3(2H)-one,
7-hydroxy-2-(2-pyridyl)-1, 2-benzisoselenazol-3(2H)-one,
7-hydroxy-2-(3-pyridyl)-1, 2-benzisoselenazol-3(2H)-one,
7-hydroxy-2-(4-pyridyl)-1, 2-benzisoselenazol-3(2H)-one,
2-(3-hydroxy-2-pyridyl)-1, 2-benzisoselenazol-3(2H)-one,
2-(2-chloro-3-pyridyl)-1, 2-benzisoselenazol-3(2H)-one,
2-(5-chloro-2-pyridyl)-1, 2-benzisoselenazol-3(2H)-one,
2-(6-methoxy-3-pyridyl)-1, 2-benzisoselenazol-3(2H)-one,
2-(2, 6-dichloro-3-pyridyl)-1, 2-benzisoselenazol-3(2H)-one,
2-(4, 6-dimethyl-2-pyridyl)-1, 2-benzisoselenazol-3(2H)-one,
2-(6-hydroxy-2-pyridyl)-1, 2-benzisoselenazol-3(2H)-one,
2-(3-nitro-2-pyridyl)-1, 2-benzisoselenazol-3(2H)-one,
2-(5-nitro-2-pyridyl)-1, 2-benzisoselenazol-3(2H)-one,
6-methyl-2-(2-pyridyl)-1, 2-benzisoselenazol-3(2H)-one,
6-methoxy-2-(2-pyridyl)-1, 2-benzisoselenazol-3(2H)-one,
5-nitro-2-(2-pyridyl)-1, 2-benzisoselenazol-3(2H)-one,
7-methoxy-2-(2-pyridyl)-1, 2--benzisoselenazol-3(2H)- one,
6, 7-methylenedioxy-2-(2-pyridyl)-1, 2-benzisoselenazol-3(2H)-one,
2-(3-methyl-2-pyridyl)-1, 2-benzisoselenazol-3(2H)-one,
2-(4-methyl-2-pyridyl)-1, 2-benzisoselenazol-3(2H)-one,
2-(5-methyl-2-pyridyl)-1, 2-benzisoselenazol-3(2H)-one,
2-(6-methyl-2-pyridyl)-1, 2-benzisoselenazol-3(2H)-one,
2-(3, 5-dichloro-2-pyridyl)-1, 2-benzisoselenazol-3(2H)-one,
2-(4-chloro-2-pyridyl)-1, 2-benzisoselenazol-3(2H)-one,
2-(4-carboxyl-5-chloro-2-pyridyl)-1, 2-benzisoselenazol-3(2H)-one,
2-(3-carboxyl-2-pyridyl)-1, 2-benzisoselenazol-3(2H)-one,
2-(2-tetrahydropyridyl)-1, 2-benzisoselenazol-3(2H)-one,
2-(2-pyrimidinyl)-1, 2-benzisoselenazol-3(2H)-one,
2-(2-thiazolyl)-1, 2-benzisoselenazol-3(2H)-one,
2-(benzothiazolyl)-1, 2-benzisoselenazol-3(2H)-one,
2-(4-methyl-2-pyrimidinyl)-1, 2-benzisoselenazol-3(2H)-one,
2-(5-nitro-2-pyrimidinyl)-1, 2-benzisoselenazol-3(2H)-one,
2-(4, 6-dimethyl-2-pyrimidinyl)-1, 2-benzisoselenazol-3(2H)-one,
(2, 6-dimethyl-4-pyrimidinyl)-1, 2-benzisoselenazol-3(2H)-one,
2-(5-ethoxy-2-ethylthio-4-pyrimidinyl)-1, 2-benzisoselenazol-3(2H)-one,
2-(5-ethoxycarbonyl-2-hydroxy-4-pyrimidinyl)-1, 2-benzisoselenazol-3(2H)-one,
2-(5-carboxyl-4-pyrimidinyl)-1, 2-benzisoselenazol-3(2H)-one,
2-(6-chloro-2-methylthio-4-pyrimidinyl)-1, 2-benzisoselenazol-3(2H)-one,
2-(4-chloro-2-methylthio-6-pyrimidinyl)-1, 2-benzisoselenazol-3(2H)-one,
2-(4-chloro-6-methyl-2-pyrimidinyl)-1, 2-benzisoselenazol-3(2H)-one,
2-(6-chloro-3-nitro-2-pyrimidinyl)-1 , 2-benzisoselenazol-3(2H)-one,
2-(2-chloro-5-nitro-4-pyrimidinyl)-1, 2-benzisoselenazol-3(2H)-one,
2-(4, 6-dichloro-2-pyrimidinyl)-1, 2-benzisoselenazol-3(2H)-one,
2-(4, 6-dichloro-5-pyrimidinyl)-1, 2-benzisoselenazol-3(2H)-one,
2-(4, 6-dihydroxy-2-pyrimidinyl)-1, 2-benzisoselenazol-3(2H)-one,
2-(2, 6-dihydroxy-4-pyrimidinyl)-1, 2-benzisoselenazol-3(2H)-one,
2-(2, 4-dihydroxy-5-pyrimidinyl)-1, 2-benzisoselenazol-3(2H)-one,
2-(4, 6-dithio-2-pyrimidinyl)-1, 2-benzisoselenazol-3(2H)-one,
2-(6-hydroxy-2-thio-4-pyrimidinyl)-1, 2-benzisoselenazol-3(2H)-one,
2-(6-hydroxy-2-methylmercapto-4-pyrimidinyl)-1, 2-benzisoselenazol-3 (2H)- one,
2-(4-hydroxy-6-methyl-2-pyrimidinyl)-1, 2-benzisoselenazol-3(2H)-one,
2-(2-hydroxy-5-methyl-4-pyrimidinyl)-1, 2-benzisoselenazol-3(2H)-one,
2-(1-benzopyrazolo(3, 4-d)pyrimidin-4-yl)-1, 2-benzisoselenazol-3(2H)-one,
2-(pyrazolo(3, 4-d)pyrimidin-4-yl)-1, 2-benzisoselenazol-3(2H)-one,
2-(6-hydroxypyrazolo(3,4-d)pyrimidin-4-yl)-1, 2-benzisoselenazol-3(2H)-one,
2-(6-thiopyrazolo(3, 4-d)pyrimidin-4-yl)-1, 2-benzisoselenazol-3(2H)-one,
2-(2-hydroxy-4-pyrimidinyl)-1, 2-benzisoselenazol-3(2H)-one,
2-(4-chloro-2-benzothiazolyl)-1, 2-benzisoselenazol-3(2H)-one,
2-(4-methoxy-2-benzothiazolyl)-1, 2-benzisoselenazol-3(2H)-one,
2-(6-methoxy-2-benzothiazolyl)-1, 2-benzisoselenazol-3(2H)-one,
2-(4-methyl-2-benzothiazolyl)-1, 2-benzisoselenazol-3(2H)-one,
2-(2-methyl-5-benzothiazolyl)-1, 2-benzisoselenazol-3(2H)-one,
2-(6-nitro-2-benzothiazolyl)-1, 2-benzisoselenazol-3(2H)-one,
2-(6-bromo-2-benzothiazolyl)-1, 2-benzisoselenazol-3(2H)-one,
2-(5, 6-dimethyl-2-benzothiazolyl)-1, 2-benzisoselenazol-3(2H)-one,
2-(6-ethoxy-2-benzothiazolyl)-1, 2-benzisoselenazol-3(2H)-one,
6-methyl-2-(2-thiazolyl)-1, 2-benzisoselenazol-3(2H)-one,
6-chloro-2-(2-thiazolyl)-1, 2-benzisoselenazol-3(2H)-one,
6-methoxy-2-(2-thiazolyl)-1, 2-benzisoselenazol-3(2H)-one,
5-nitro-2-(2-thiazolyl)-1, 2-benzisoselenazol-3(2H)-one,
5-chloro-2-(2-thiazolyl)-1, 2-benzisoselenazol-3(2H)-one,
7-methoxy-2-(2-thiazolyl)-1, 2-benzisoselenazol-3(2H)-one,
6, 7-methylenedioxy-2-(2-thiazolyl)-1, 2-benzisoselenazol-3(2H)-one,
2-(4-methyl-2-thiazolyl)-1, 2-benzisoselenazol-3(2H)-one,
2-(5-nitro-2-thiazolyl)-1, 2-benzisoselenazol-3(2H)-one,
2-(2-thiazolinyl)-1, 2-benzisoselenazol-3(2H)-one,
2-(5-chloro-2-thiazolyl)-1, 2-benzisoselenazol-3(2H)-one,
2-(3-methyl-5-isothiazolyl)-1, 2-benzisoselenazol-3(2H)-one,
2-(2-imidazolyl)-1, 2-benzisoselenazol-3(2H) one,
2-(5-ethoxycarbonyl-2-imidazolyl)-1, 2-benzisoselenazol-3(2H)-one,
2-(3-pyrazolyl)-1, 2-benzisoselenazol-3(2H)-one,
2-(4-cyano-5-pyrazolyl)-1, 2-benzisoselenazol-3(2H)-one,
2-(4-ethoxycarbonyl-3-pyrazolyl)-1, 2-benzisoselenazol-3(2H)-one,
2-(4-ethoxycarbonyl-2-phenyl-3-pyrazolyl)-1, 2-benzisoselenazol-3(2H)-one,
2-(4-cyano-3-pyrazolyl)-1, 2-benzisoselenazol-3(2H)-one,
2-(2-phenyl-3-pyrazolyl)-1, 2-benzisoselenazol-3(2H)-one,
2-(1-phenyl-5-pyrazolyl)-1, 2-benzisoselenazol-3(2H)-one,
2-(1-phenyl-5-pyrazolinon-3-yl)-1, 2-benzisoselenazol-3(2H)-one,
2-(5-hydroxy-3-pyrazolyl)-1, 2-benzisoselenazol-3(2H)-one,
2-(1, 3, 4-thiadiazolyl-2-yl)-1, 2-benzisoselenazol-3(2H)-one,
2-(5-thio-1, 3, 4-thiadiazolyl-2-yl)-1, 2-benzisoselenazol-3(2H)-one,
2-(5-methyl-1, 3, 4-thiadiazolyl-2-yl)-1, 2-benzisoselenazol-3(2H)-one,
2-(5-trifluoromethyl-1, 3, 4-thiadiazolyl-2-yl)-1, 2-benzisoselenazol-3(2H)-one,
2-(5-tert-butyl-1, 3, 4-thiadiazolyl-2-yl)-1, 2-benzisoselenazol-3(2H)-one,
2-(2-pyrazinyl)-1, 2-benzisoselenazol-3(2H)-one,
2-(2-carboxyl-3-pyrazinyl)-1, 2-benzisoselenazol-3(2H)-one,
2-(2-pyridazinyl)-1, 2-benzisoselenazol-3(2H)-one,
2-(2-benzimidazolyl)-1, 2-benzisoselenazol-3(2H)-one,
2-(5, 6-dimethyl-2-benzimidazolyl)-1, 2-benzisoselenazol-3(2H)-one,
2-(5-benzotriazolyl)-1, 2-benzisoselenazol-3(2H)-one,
2-(7-chloro-1, 2, 4-benzotriazinyl-3-yl)-1, 2-benzisoselenazol-3(2H)-one,
2-(1, 2, 4-benzotriazinyl-3-yl)-1, 2-benzisoselenazol-3(2H)-one,
2-(7-bromo-1, 2, 4-benzotriazinyl-3-yl)-1, 2-benzisoselenazol-3(2H)-one,
2-(7-fluoro-1, 2, 4-benzotriazinyl-3-yl)-1, 2-benzisoselenazol-3(2H)-one,
2-(7-nitro-1, 2, 4-benzotriazinyl-3-yl)-1, 2-benzisoselenazol-3(2H)-one,
2-(2-benzothienyl)-1, 2-benzisoselenazol-3(2H)-one,
2-(3-benzothienyl)-1, 2-benzisoselenazol-3(2H)-one,
2-(2-thienyl)-1, 2-benzisoselenazol-3(2H)-one,
2-(2, 1, 3-benzothiadiazolyl-4-yl)-1, 2-benzisoselenazol-3(2H)-one,
2-(1, 2, 4-triazinyl-4-yl)-1, 2-benzisoselenazol-3(2H)-one,
2-(2, 6-dithio-1, 3, 5-triazinyl-4-yl)-1, 2-benzisoselenazol-3(2H)-one,
2-(5, 6-dimethyl-1, 2, 4-triazinyl-3-yl)-1, 2-benzisoselenazol-3(2H)-one,
2-(5, 6-diphenyl-1, 2, 4-triazinyl-3-yl)-1, 2-benzisoselenazol-3(2H)-one,
2-(1, 2, 4-triazolyl-4-yl)-1, 2-benzisoselenazol-3(2H)-one,
2-(5-thio-1, 2, 4-triazolyl-3-yl)-1, 2-benzisoselenazol-3(2H)-one,
2-(5-tetrazolyl)-1, 2-benzisoselenazol-3(2H)-one,
2-(5-quinolinyl)-1, 2-benzisoselenazol-3(2H)-one,
2-(2-methyl-4-quinolinyl)-1, 2-benzisoselenazol-3(2H)-one,
2-(6-nitro-5-quinolinyl)-1, 2-benzisoselenazol-3(2H)-one,
2-(1, 2, 3, 4-tetrahydroquinolin-8-yl)-1, 2-benzisoselenazol-3(2H)-one,
2-(2-quinolinyl)-1, 2-benzisoselenazol-3(2H)-one,
2-(3-quinolinyl)-1, 2-benzisoselenazol-3(2H)-one,
2-(8-quinolinyl)-1, 2-benzisoselenazol-3(2H)-one,
2(6-methoxy-8-quinolinyl)-1, 2-benzisoselenazol-3(2H)-one,
2-(1-isoquinolinyl)-1, 2-benzisoselenazol-3(2H)-one,
2-(5-isoquinolinyl)-1, 2-benzisoselenazol-3(2H)-one,
2-(5-indolyl)-1, 2-benzisoselenazol-3(2H)-one,
2-(5-isoindolyl)-1, 2-benzisoselenazol-3(2H)-one,
2-(5-indazolyl)-1, 2-benzisoselenazol-3(2H)-one,
2-(6-chloro-3-indazolyl)-1, 2-benzisoselenazol-3(2H)-one
2-(6-indazolyl)-1, 2-benzisoselenazol-3(2H)-one,
2-(7-indazolyl)-1, 2-benzisoselenazol-3(2H)-one,
2-(9-ethyl-3-carbazolyl)-1, 2-benzisoselenazol-3(2H)-one,
2-[(3, 4-dihydroimidazol-2-yl)-2-ethenyl]-1, 2-benzisoselenazol-3(2H)-one
2-(2-pyridylmethyl)-1, 2-benzisoselenazol-3(2H)-one,
2-(2-furylmethyl)-1, 2-benzisoselenazol-3(2H)-one,
2-[4-(2-hydroxyimino-ethyl)]-1, 2-benzisoselenazol-3(2H)-one
2-[4-(7-n-butyl-benzo(1, 2-c-dihydrofuran-2-one))]-1, 2-benzisoselenazol-3 (2H)-one.

The present invention also provides a pharmaceutical composition, comprising a therapeutically effective amount of one or more of the compounds of formula (I) and/or the stereo isomer thereof, and a conventional pharmaceutically acceptable excipient or carrier.

The combination may be prepared according to the known methods in the art. For this purpose, a compound of the general formula (I) and/or its stereo isomer is mixed with one or more solid or liquid pharmaceutically acceptable excipient and/or carrier to obtain a suitable administration or a dosage form suitable for human or veterinary use.

The compounds of the present invention or the composition containing the compounds can be administered in dosage forms through oral or parenteral route, such as oral, intravenous, intramuscular, subcutaneous, nasal, tunica mucosa oris, transdermal, intraperitoneal or rectal. The dosage forms may be tablets, capsules, pills, aerosol, pellets, powder, solution, suspension, emulsion, granule, suppository or lyophilized powder, etc. The formulation may be a conventional form, a sustained release form, a controlled release form or various microgranule delivery systems.

Various carriers commonly used in the art can be used to prepare tablets. Carriers such as, diluents and absorbents, for example, starch, dextrin, calcium sulfate, lactose, mannitol, sucrose, sodium chloride, glucose, urea, calcium carbonate, China clay, microcrystallinecellulose, aluminum silicate, etc; moistening agents and adhesives, for example, water, glycerol, polyethylene glycol, ethanol, propanol, starch paste, dextrin, syrup, honey, glucose solution, Arabia gum, gelatin liquid, carboxymethylcellulose sodium, lac, methylcellulose, potassium phosphate, polyvinylpyrrolidone, etc; disintegrants, for example, dry starch, alginate, agar powder, algin starch, sodium bicarbonate and citric acid, calcium carbonate, polyethylenesorbitol fatty acid ester, sodium dodecanylsulfonate, methylcellulose, ethylcellulose, etc; disintegration inhibitors, for example, sucrose, glycerin tristearate, cocoa butter, hydrogenated oils, etc; sorbefacients, for example, quaternary ammonium salts, sodium dodecanylsulfonate, etc; lubricants, for example, talc, silica, corn starch, stearates, boric acid, liquid paraffin, polyethylene glycol, etc.

The tablets can be further coated, for example sugar-coated, thin film-coated, enteric-coating coated, or two-layered or multi-layered tablets.

Carriers commonly used in the art can be used for the preparation of pellets from dosage units, such as, diluents and absorbants, for example, glucose, lactose, starch, cocoa butter, hydrogenated vegetable oil, polyvinylpyrrolidone, Gelucire, Kaolin, talc, etc; adhesives, for example, Arabia gum, tragacanth gum, gelatin, ethanol, honey, sugar liquid, rice paste or flour paste, etc; disintegrants, for example, agar powder, dry starch, alginate, sodium dodecanylsulfonate, methylcellulose, ethylcellulose, etc.

Carriers commonly used in the art can be used for the preparation of suppositories from dosage units, for example, polyethylene glycol, lethitin, cocoa butter, higher alcohols and their esters, gelatin and semi-synthetic glycerol esters, etc.

To prepare capsules from dosage units, the compounds of the invention or their stereo isomers are admixed with the above-mentioned carriers and the mixtures are fixed into hard gelatin capsules or soft capsules. The active ingredient compounds of general formula (I) or their stereo isomers can be encapsuled into microcapsules, or suspended an aqueous medium to prepare a suspension, or be fixed into hard capsules or prepared into injections.

To produce injections such as solution, emulsion, lyophilized powder for injection and suspensions from dosage units, all the common used diluents known in the art can be used, such as, water, ethanol, polyethylene glycol, trimethylene glycol, ethoxylated isostearol, multioxygenated isostearol, polyethyleneoxide sorbitol alkanates. In addition, an appropriate amount of sodium chloride, glucose or glycerol can be added into injections to produce isotonic injections. Moreover, the conventional solubilizing agents, buffer, and pH modulator can also be added.

In addition, if necessary, colorants, antiseptics, fragrants, flavoring agents, sweetening agents and the other materials can be added into the dosage forms.

The administration dosage of the compound of general formula (I) or its stereo isomer of the invention varies, depending on several factors, for example, the characters and severeness of disorders to be prevented or treated, gender, age, weight, and individual reaction of patients, on the concrete conpound, administration route and frequency, etc. Generally, for a patient with a body weight of about 75kg, it is preferred to administer once daily or severally divided (such as twice, three time or four times) dosages in an amount of from 0.001mg/kg to 100 mg/kg body weight per day, preferably from 0.01 mg/kg to 20 mg/kg body weight per day, and more preferably from 0.1mg/kg to 5 mg/kg body weight per day.

### Brief Description of the Drawings

Fig 1. Effect of Amiloride on the forward and backward Na⁺/Ca²⁺ exchange current in guinea pigs ventricular myocardium.
Fig 2. Effect of KB-R7943 on the forward and backward Na⁺/Ca²⁺ exchange current in guinea pigs ventricular myocardium.
Fig 3. Effect of IMM-001 on the forward and backward Na⁺/Ca²⁺ exchange current in guinea pig ventricular myocardium.
Fig 4. Comparison of severity of myocardial ischemia in dogs of each group and measurement of ventricular surface electrogram.
Fig 5. Acute myocardial infarction area in dogs of each group.
Fig 6. Comparison of serum creatine kinase (CK) in dogs of each group.
Fig 7. Comparison of serum lactate dehydrogenase (LDH) in dogs of each group.
Fig 8. Comparison of serum glutamic oxalacetic transaminase (AST) in dogs of each group.
Fig 9. Comparison of coronary blood flow in dogs of each group.
Fig 10. Comparison of blood oxygen in dogs of each group.
Fig 11. Effects of IMM-001 on the left ventricular systolic pressure (LVSP) of myocardial ischemia/reperfusion rats.
Fig 12. Effects of IMM-001 on the left ventricular end diastolic pressure (LVSEDP) of myocardial ischemia/reperfusion rats.
Fig 13. Effects of IMM-001 on the left arterial systolic pressure (SBP) of myocardial ischemia/reperfusion rats.
Fig 14. Effects of IMM-001 on the maximum of intraventricular pressure velocity (±dp/dtmax) of myocardial ischemia/reperfusion rats.
Fig 15. Effects of IMM-001 on the left mean arterial pressure (MAP) of myocardial ischemia/reperfusion rats.

### Examples

The following examples are intended to illustrate the invention, but these examples are intended to illustrate the invention and not be construed to limit the scope of the invention.

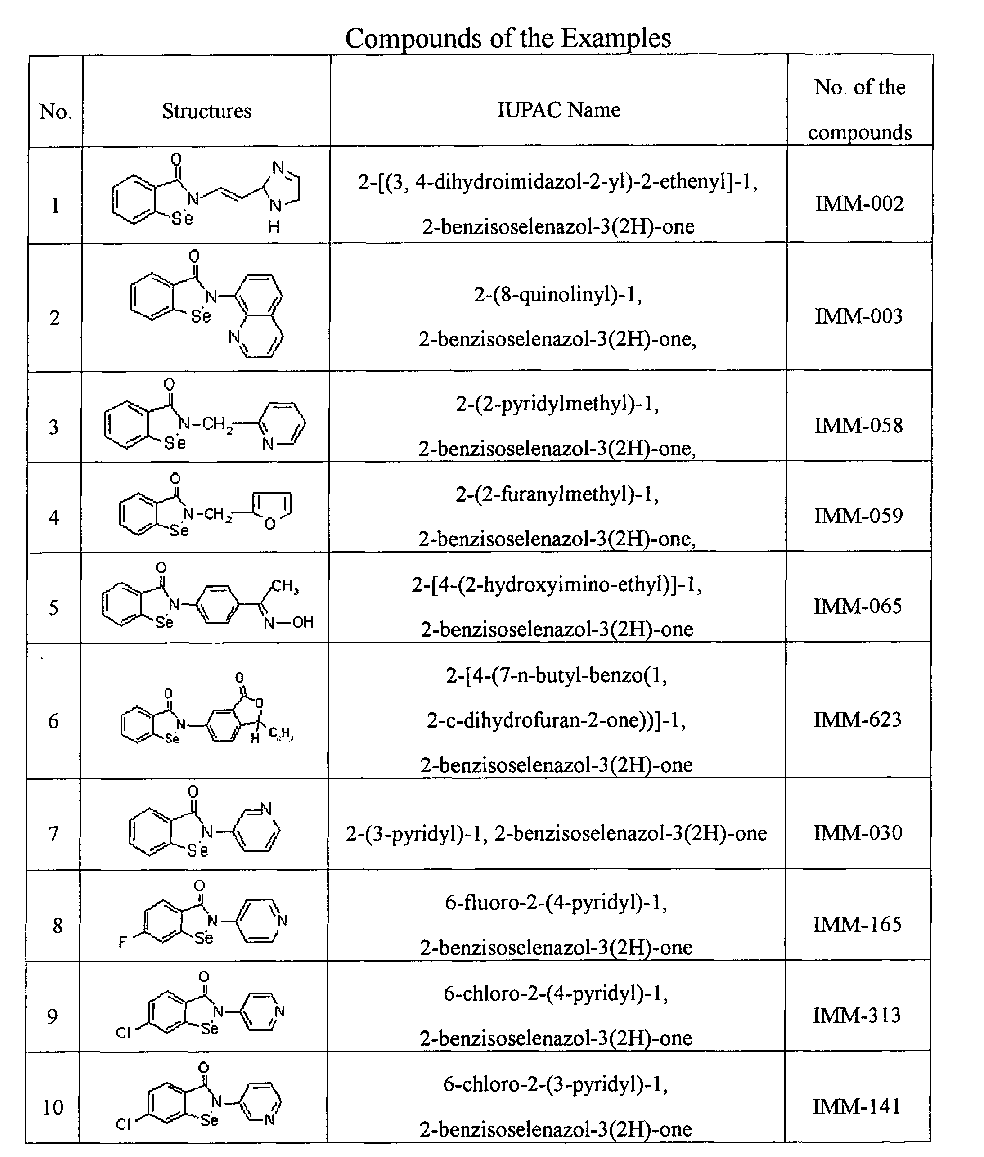

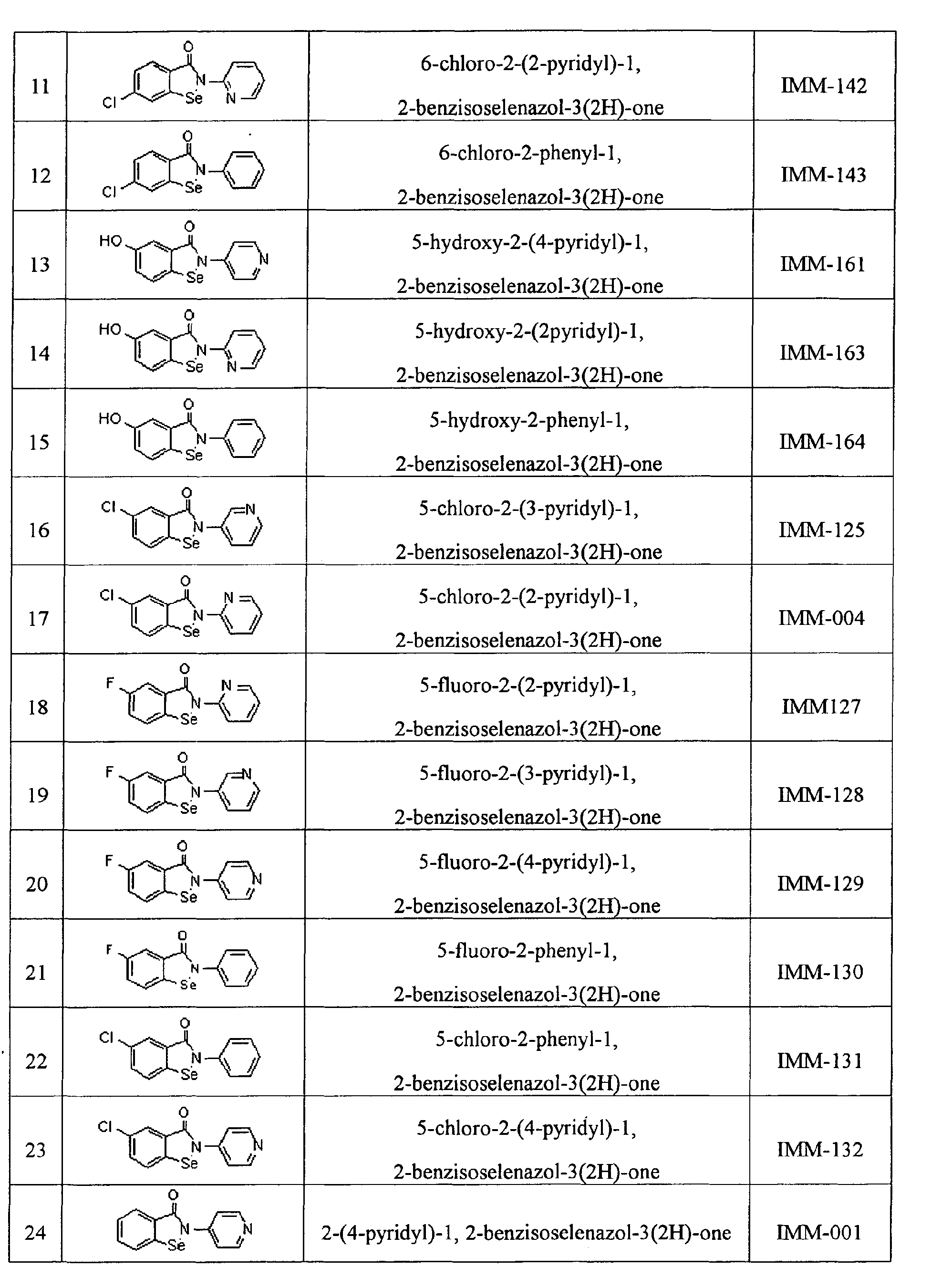

The Na⁺/Ca²⁺ exchanger is an important biphasical ion transport protein locating on the excitatory cell membrane, ofwhich the principle function is to extrude the Ca²⁺ ion entered during the exciting stage and restore the intracellular calcium to an inactivate level. During the Na⁺/Ca²⁺ exchange, three Na⁺ are transported into the cell per extrusion of each Ca²⁺, thus there is a net charge cross-membrane movement which induces a cross-membrane current. At the early stage of myocardial ischemia-reperfusion, the cell membrane injury results in the abnormality of membrane potential and an alteration of membrane permeability, a large amount of Na⁺ enters into the cell, and the Na⁺/Ca²⁺ exchanger is reverse-activated to extrude excessive Na⁺ and results in intracellular Ca²⁺ overload, which exacerbates injury and death of the cells. Hence, the Na⁺/Ca²⁺ reverse-exchanger is deemed to be an important mechanism of the adverse effects of ischemia/reperfusion. Up to now, there is no drug that can specifically block the Na⁺/Ca²⁺ reverse-exchange all over the world. The cell level pathological model based screening system we adopted and the relevant *in vitro and in* vivo studies, have provided theoretical and practical guidelines for the development of guidance compounds targeted at Na⁺/Ca²⁺ exchanger, the potential and important target for drug action, and is also beneficial to the development of drugs against myocardial cell insults.

### 1. Establishment of assaying method for biphasical current detection in Na⁺/Ca²⁺ exchange system

Since the reverse mode of Na⁺/Ca²⁺ exchange process is one of the main mechanisms involving in ischemia/reperfusion insult, it is of great importance to establish cell model represented reverse mode Na⁺/Ca²⁺ exchange process for the development of drugs against ischemic injury. Therefore, we successfully established cell model of reverse mode Na⁺/Ca²⁺ exchange system by intracellular Na⁺ dialysis technique, and recorded Na⁺/Ca²⁺ exchange currents by patch clamp experiment.

Internal solution contained certain concentration of NaCl was filled to the microcatheter, and ion exchange process between the cellular and internal solutions was conducted after whole cell currents were recorded by patch clamp. Na⁺ contained in the internal solution was dialyzed into the cell; result in the high intracellular Na⁺ concentration. After that, the cell was champed at a holding potential of -40 mV, then depolarized to +60mV and followed by a conversely declining ramp stimuli at a speed of 80 mV·s⁻¹ and 2s conditioning pulse, then repolarized to -100mV with a frequency of 0.05 Hz. Under the stimulation protocol, current from outward to the inward direction was recorded by perfusing modified biphasical Tyrode solution (in which the outward and inward currents can be recorded simultaneously) with the addition of 1mmol·L⁻¹ of BaCl₂ and 2mmol·L⁻¹ of CsCl to block K⁺ channels, 1µmol·L⁻¹ of verapamil to block L-type Ca²⁺ channels, and 20µmol·L⁻¹ of ouabain to block Na⁺/K⁺ ATPase, and 20mmol·L⁻¹ of TEA to block potassium channels. The recorded inward currents were presented in a linear manner owing to the blockade of other time-dependent currents. With the increase of intracellular Na⁺ level and prolongation of time, the reverse mode of Na⁺/Ca²⁺ exchange process was activated. As a result, the outward currents were significantly augmented. Comparative study with different concentration of intracellular Na⁺ (0, 15, 25 and 35mmol·L⁻¹) subjected to the experiment revealed that 25mmol·L⁻¹ NaCl was suitable to conduct the dialysis experiment, and applicable in the assay method. Under such experimental conditioning, the maximum reverse mode current was recorded 3 min after cell crushed. No significant "run-down" was observed within the record period of 8 minutes. The inward and outward current were inhibited by 5mmol·L⁻¹ of Ni²⁺ treatment, and the experiment result in two current-voltage curves in the absence or presence of Ni²⁺. The Ni²⁺ sensitive current, also called electrophysiological Na⁺/Ca²⁺ exchange current (I_{Na-Ca}), was the minus result of two current-voltage curves. The reverse mode potential of I_{Na-Ca} was about -20mV

Screening studies were performed to the compounds synthesized of the present invention with the above electrophysiological model. New compound named IMM-001 shows selectively inhibitory effect on reverse mode of Na⁺/Ca²⁺ exchange induced by intracellular Na⁺ overload. The inhibitory effect was more potent than the ordinary used Na⁺/Ca²⁺ exchange blocker, Amiloride. The result also demonstrates that the effective concentration of Amiloride is above 10⁻⁵mol·L⁻¹, and become evident at 10⁻⁴mol·L⁻¹, while IMM-001 in 10⁻⁷ to 10⁻⁵ mol·L⁻¹ shows significant inhibitory effect.

A compound KB-R7943 derived from Isothiourea was reported to be a selective and potent inhibitor of Na⁺/Ca²⁺ exchange process in 1996. Since then, KB-R7943 has been regarded as the newest and specific blocker on reverse mode of Na⁺/Ca²⁺ exchange process and widely used in pharmacological studies. However, the ion conditioning of the experiment was under the control, that merely one of inward and outward current can be recorded simultaneously. Thus, the unidirectional ion conditioning does not fit in with physiological and pathological abnormalities, that is, the inward and outward current could be observed simultaneously. The inhibitory effect of KB-R7943 on I_{Na-Ca} via above model, the result revealed that KB-R794 demonstrated significant inhibitory effect on reverse mode of Na⁺/Ca²⁺ exchange process while the obverse mode of Na⁺/Ca²⁺ exchange process was also inhibited at the same time. The observation shows the poor selectivity of KB-R794 on Na⁺/Ca²⁺ exchange system.

On the basis of above observations, more specific and selective blocker on the reverse mode of Na⁺/Ca²⁺ exchange process was developed, while IMM-001 being a leading compound.

### 2. Procedures:

### 2.1 Preparation of single ventricular myocytes from guinea pigs

Male guinea pigs weighting 250~280g provided by the Animal Center of the Chinese Academy of Medical Sciences were hammered and sacrificed. The chest was opened and the heart was removed quickly. Fatty tissue and pericardium were removed in a Ca²⁺-free solution at 4°C, and heart was sequentially mounted on a Langendorff apparatus. First, the heart was perfused conversely via the aorta for 6 min with Ca²⁺-free solution in order to wash out residual blood, then the heart expands and the color of it turns to pink. Then the heart was perfused with Ca²⁺-free solution containing 0.1mg/ml of pronase E, 0.5 mg/ml defatted bovine serum albumin (BSA) and 150 µM Ca²⁺ for 2~3 min, until the heart expanded and became soft. The atrium was removed, and the digested heart was minced with scissors, stirred in a beaker at 37°C for 5~10min. The supernatant was diluted with Ca²⁺-free solution containing 0.5 mg/ml BSA and 1.8 mM Ca²⁺. The supernatant was placed at room temperature (20~24 °C) for 1 hr and then subjected to the experiment. During the whole perfusion period, pressure was kept at 70 cmH₂O and the temperature of solution was kept at 37 °C with continuously gassed with 95 % O₂ and 5 % CO₂.

### 2.2 Whole-Cell Patch-clamp techniques

Rod-shaped cells with clear striations and intact membrane were used. Isolated single ventricular myocytes were placed in the recording chamber (1 ml in volume), which was mounted horizontally on an inverted microscope (IMT-2, Olympus, Japan) linked with a micromanipulator (MO-303), and perfused with Tyrode solution at a flow rate of 1~2 ml/min. A suction pipette was made of glass capillaries (Shanghai Cerebral Institute, Academia Sinica) and pulled in a microelectrode puller (PP-83) via a two-stage preparation method. The pipette tip resistance was 2-4 MΩ when filled with the pipette solution. During the whole patch-clamp procedure, the stimulating pulse and signal were recorded and amplified by a patch-clamp amplifier under software control of pCLAMP5.5.1 software (Axon Instrument), and converted by AD/DA transition plate to a computer.

Cells were clamped at a holding potential of -40mV, then depolarized to +60mV and followed by a conversely declining ramp stimuli at a speed of 80 mV·s⁻¹ and 2s conditioning pulse, then repolarized to -100mV with a frequency of 0.05 Hz. The currents (*I*_{*Na-Ca*}) were recorded during the experiment. Because the declining ramp pulse is a kind of slow depolarizing voltage ramps, it is not necessary to compensate the series resistance and membrane capacity. In order to block the interference induced by other time-dependent currents, the pipette solution containing 20µmol·L⁻¹ of ouabain to block Na⁺/K⁺ ATPase, 1mmol·L⁻¹ of BaCl₂ and 2mmol·L⁻¹ of CsCl to block K⁺ channels, 1µmol·L⁻¹ of verapamil to block L-type Ca²⁺ channels, and 20mmol·L⁻¹ of TEA to block potassium channels. According to the Fabiato & Fabiato equation, intracellular free Ca²⁺ concentration, 153 nM, was calculated from the Ca²⁺ level of internal solution contained high level of EGTA (42mM) and CaCl₂ (29mM). The currents produced by biphasically Na⁺/Ca²⁺ exchange process were specifically inhibited by extracellular high concentration of Ni²⁺ (2 or 5mM). The Ni²⁺ sensitive current, also called electrophysiological Na⁺/Ca²⁺ exchange current (I_{Na-Ca}), was the minus result of two current-voltage curves. In order to exclude the influence of different cells size on the recorded value of currents, the current value was represented as the current density (pA/pF). The whole-cell voltage clamp procedure was carried out at 31~32°C.

### 2.3 Reagents

Ca²⁺-free solution (mM): NaCl 90, KCl 10, KH₂PO₄ 1.2, MgSO₄ 5, NaHCO₃ 15, taurine 30, glucose 20, adjusting pH to 7.4 by 1N NaOH

Normal Tyrode's solution (mM): NaCl 150, KCl 5.4, MgCl₂ 2, CaCl₂ 1.8, HEPES 5, Glucose 10, adjusting pH to 7.35 by 1N NaOH

Tyrode solution (mM) for recording Na⁺/Ca²⁺ exchange current: NaCl 140, MgCl₂ 2, CaCl₂ 1.8, HEPES 5, Glucose 10, adjusting pH to 7.4 by 1N NaOH

Normal pipette solution (mM): KCl 140, MgCl₂ 0.5, EGTA 10, HEPES 10, adjusting pH to 7.2 by 1N KOH

Pipette solution (mM) for recording Na⁺/Ca²⁺ exchange current: EGTA 42, CaCl₂ 29, MgCl₂ 13, Aspartic acid 42, TEA 20, HEPES 5, Na₂ATP 5, adjusting pH to 7.4 by 1N CsOH. During experiment, adding into NaCl 5, 15 and 25 mM respectively according to different concentration of Na⁺ in the pipette solution that the concentration of Na⁺ to 15, 25 and 35mM

All solutions were prepared by double distilled water. Ca²⁺ free solution and Tyrode soltion were prepared freshly before experiments. The pipette solution was filtered by 0.22 µm microfilter after preparation, packed with Eppendorf pipe and strored below 0°C.

### 2.4 Statistical Analysis

The original current graphs were measured and analyzed with pCLAMP 5.5.1 and pCLAMP 6.0.1 softwares. All the values were presented as Mean ± SE, n is the number of cells; paired and student's *t* test were used for the statistical analysis. P values of less than 0.05 were considered being significant.

### Screening studies on cells

### 1. Recording of normal Na⁺/Ca²⁺ exchange current

Cells were clamped at a holding potential of -40mV, then depolarized to +60mV and followed by a conversely declining ramp stimuli at a speed of 80 mV·s⁻¹ and 2s conditioning pulse, then repolarized to -100mV with frequency of 0.05 Hz. The current-voltage curve (*I-V* curve) was obtained during the experiment. Because of the blockade of other time-dependent currents, the recorded currents were almost presented in a linear manner. The inward and outward current were significantly inhibited by 5 mmol·L⁻¹ extracelluluar Ni²⁺ treatment, and the experiment result in two current-voltage curves in the absence or presence of Ni²⁺. The Ni²⁺ sensitive current, also called electrophysiological Na⁺/Ca²⁺ exchange current (I_{Na-Ca}), was the minus result of two current-voltage curves.

### 2. Comparative study of Amiloride, KB-R7943 and IMM-001 on Na⁺/Ca²⁺ exchange current inhibition

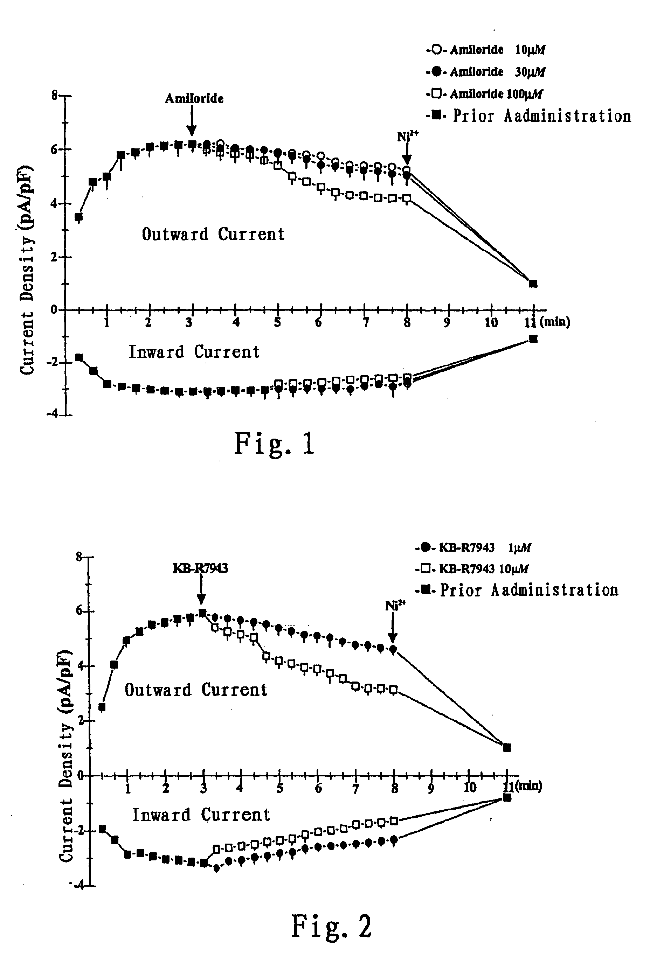

Comparative study was performed by evaluating the inhibitory potential of Amiloride (blocker of Na⁺/H⁺ exchange), 2-[2-[4-(4-nitrobenzyl oxy)phenyl]]isothiourea (KB-R7943; inhibitor of reverse mode Na⁺/Ca²⁺ exchange and often used as a pharmacological tool recently) and a new compound (IMM-001) on currents produced by Na⁺/Ca²⁺ exchange process. The inhibitory ratio of Amiloride on *I*_{Na-Ca} at +50 mV was 15 %, 23 % and 41 % at the concentration of 10, 30 and 100 µM respectively; the inhibitory ratio of Amiloride on *I*_{Na-Ca} at -80 mV was 6 %, 15 % and 23 % respectively. The result indicated that the inhibitory effect of Amiloride on outward *I*_{Na-Ca} was more potent that that of inward *I*_{Na-Ca}. The inhibitory ratio of KB-R7943 on *I*_{Na-Ca} at +50 mV was 29 % and 61 % at the concentration of 1 and 10 µM respectively; the inhibitory ratio of KB-R7943 on *I*_{Na-Ca} at -80 mV was 22 and 57 % respectively. The result indicated the inhibitory effect of KB-R7943 on inward/outward *I*_{Na-Ca} without selectivity under biphasic ion conditioning. The inhibitory ratio of IMM-001 on *I*_{Na-Ca} at +50 mV was 28 %, 48 % and 66 % at the concentration of 0.1, 1 and 10 µM respectively; the inhibitory ratio of IMM-001 on *I*_{Na-Ca} at -80 mV was 15 %, 24 % and 42 % respectively. The inhibitory effect of IMM-001 on outward *I*_{*Na-Ca*} was more potent than that of inward *I*_{*Na-Ca*}. Above evidence demonstrated that Amiloride at low concentration has little effect on inward and outward *I*_{*Na-Ca*}; KB-R7943 showed inhibitory effect on both inward and outward currents simultaneously, and has significant influence on normal membrane function of extruding Ca²⁺. IMM-001 is the most potent inhibitor among three compounds, and its inhibitory effect on outward current with selectivity. Compared to other compounds, IMM-001 act on specific target with high selectivity, which is beneficial to the inhibition of intracellular Ca²⁺ overload induced by isochemia/perfusion injury, and to the recovery of myocytes damage caused by intracellular Ca²⁺ overload. (see Fig. 1, Fig. 2 and Fig. 3)

### 3. Comparative and screening studies: inhibitory effects of 22 candidate compounds on Na⁺/Ca²⁺ exchange current

Comparing with KB-R7943 and Amiloride, the inhibitory effect of IMM-001 on outward Na⁺/Ca²⁺ exchange current was more potent and with significant selectivity. On the basis of such evidence, 22 candidate compounds, ofwhich the leading compound is IMM-001, were synthesized. The cell model of intracellular Na⁺ overload followed by the activation of I_{Na-Ca} was established via patch-clamp technique. Thus, the inhibitory effects of 22 candidate compounds on Na-Ca exchange current were evaluated, and compared with IMM-001, the positive control. The aim of current study is to find out the more potent inhibitors of outward Na⁺/Ca²⁺ exchange current, and the selectivity of their actions was more specific than IMM-001. The results are shown in the following table.

| Inhibitory effects of 22 candidate compounds on Na⁺/Ca²⁺ exchange current | | | |
|---|---|---|---|
| File number of compounds | n | Inhibitory rate of outward I_{Na-Ca} (%) | Inhibitory rate of inward I_{Na-Ca} (%) |
| IMM-002 | 5 | 32* | 34 |
| IMM-003 | 6 | 22 | ― |
| IMM-004* | 3 | 52* | 44 |
| IMM-058 | 5 | 3 | ― |
| IMM-059 | 6 | 8 | 15 |
| IMM-065* | 6 | 55* | 43 |
| IMM-623 | 4 | 11 | 15 |
| IMM-030 | 4 | 12 | 17 |
| IMM-313* | 3 | 50* | 28 |
| IMM-141 | 4 | ― | 2 |
| IMM-142 | 3 | 46 | 57 |
| IMM-143 | 3 | ― | ― |
| IMM-161 | 3 | ― | ― |
| IMM-163 | 4 | 14 | 19 |
| IMM-164 | 4 | 8 | 22 |
| IMM-125 | 3 | 35 | 14 |
| IMM-127* | 5 | 43* | 21 |
| IMM-128* | 3 | 44* | 42 |
| IMM-129* | 3 | 59* | 57* |
| IMM-130 | 3 | 30 | 43 |
| IMM-131 | 3 | 22 | 15 |
| IMM-132* | 3 | 44* | 14 |

| | | | |
|---|---|---|---|
| * *P* < 0.05 | | | |

7 compounds with more potently and selectively inhibitory effect on outward I_{Na-Ca} than that of inward current were screened out of 22 compounds by electrophysiological studies (in myocardial cells level). Those compounds showed specific inhibitory effect on outward I_{Na-Ca}.

### Example 1: In vitro studies

### (1) Inhibitory effect of the invented compound on Na⁺/Ca²⁺ exchange current in myocardial cell

Effects of above compounds on inward and outward Na⁺/Ca²⁺ exchange currents were evaluated. The tested compounds demonstrate certain inhibitory effect on reverse mode of Na/Ca exchange process. The inhibitory ratio of compounds on Na⁺/Ca²⁺ exchange current is higher than 40%; the inhibitory ratio of the most potent one is over 60%.(See table below)

| Compound | Inhibitory rate of outward I_{Na-Ca} (%) | Inhibitory rate of inward I_{Na-Ca} (%) |
|---|---|---|
| IMM-065 | 55 | 43 |
| IMM-313 | 50 | 28 |
| IMM-004 | 52 | 44 |
| IMM-127 | 43 | 21 |
| IMM-128 | 44 | 42 |
| IMM-129 | 59 | 57 |
| IMM-132 | 44 | 14 |

### (2) Protective effect of the compounds on isolated heart subjected to ischemia/reperfusion injury

On the basis of above screening results obtained from electrophysiological studies, and the result from comparative study on the research of the inhibitory effect of compounds on reverse mode Na⁺/Ca²⁺ exchange process, 7 effective compounds were subjected to *in vitro* study. Among which, IMM-313, IMM-004 and IMM-132 have structures containing chlorine; IMM-127, IMM-128 and IMM-129 have structures containing fluorine; and IMM-065 has a structure containing OH. The experiment performed in the isolated heart subjected to ischemia/reprefusion insult. Prolongation of ventricular arrhythmic, incidence of ventricular extrasystole, ventricular tachycardia/ ventricular fibrillation (VT/VF) were recorded in order to evaluate the protective actions of drugs on myocardial injury. The results are shown as follows.

| Compound (10⁻⁶M) | n | Time (min) | Incidence rate (%) | | |
|---|---|---|---|---|---|
| | | AAT | VPS | VT | VF |
| Control | 13 | 28.23±1.70 | 62 | 69 | 77 |
| IMM-313 | 10 | 1.67±0.70** | 20* | 30 | 30* |
| IMM-004 | 8 | 0.70±0.33** | 37.5 | 37.5 | 0** |
| IMM-127 | 8 | 19.72±4.42 | 50 | 62.5 | 38 |
| IMM-128 | 8 | 22.10±3.49 | 25 | 50 | 50 |
| IMM-129 | 8 | 23.67±3.87 | 37.5 | 75 | 63 |
| IMM-132 | 7 | 29.13±0.69 | 28.6 | 85.7 | 85.7 |
| IMM-065 | 10 | 13.09±3.76** | 30 | 70 | 50 |

| | | | | | |
|---|---|---|---|---|---|
| AAT: arrhythmic occurrence time VPS: ventricular premature beats VT: ventricular tachycardia VF: ventricular fibrillation *P<0.05, **P<0.01 vs. control group | | | | | |

Conclusion: IMM-313, IMM-004 and IMM-065 show significant protective effects on myocardial insults at a concentration of 10⁻⁶M.

### Example 2: In Vivo Studies

### (1) Protective Effects of the tested compounds on myocardial insults in Rats after Intravenously Injection

1) The protective effect of Verapamil on the recovery of cardiovascular function was examined in rats after intravenous (femoral vein) injection of 0.3mg/Kg body weight. Incidence of VT, VF, VE and duration of arrhythmic were all reduced, as well as latency of ventricular arrhythmic was significantly prolonged in drug-treated groups.
2) The protective effect of IMM-001 on the recovery of cardiovascular function was examined in rats after intravenous (femoral vein) injection of 3mg/Kg body weight. Incidence of VT, VF, and duration of VT, VE and ventricular arrhythmic were all reduced in drug-treated groups.
3) The protective effect of IMM-004 on the recovery of cardiovascular function was examined in rats after intravenous (femoral vein) injection of 3mg/Kg body weight. Incidence of VT, VF and VE, and duration of VT and VF were all reduced, as well as latency of ventricular arrhythmic was significantly prolonged in drug-treated groups.
4) Heart rate was irreversibly reduced within 5 min (before ligation of left anterior descending coronary artery) in 41.2% (7/17) rats intravenous (femoral vein) injected with IMM-313 (3mg/Kg). Rats with reduced heart rate died at last. There is no significant effect observed after analysis of the other valid data.
5) There is no significant effect observed in rats intravenously (femoral vein) injected with IMM-065 (3mg/Kg).
6) The protective effect of IMM-127 on the recovery of cardiovascular function was examined in rats after intravenous (femoral vein) injection of 3mg/Kg body weight. Incidence of VT and VF, and duration of VT and VF were all reduced, as well as latency of ventricular arrhythmic was significantly prolonged in drug-treated groups.

The protective effect of compounds mentioned above on myocardial damage caused by ischemia/reperfusion was observed in rats intravenously injected at 3 mg/kg body weight. Arrhythmic potency induced by myocardial insults was used to assess the potential protective effect of the compounds.

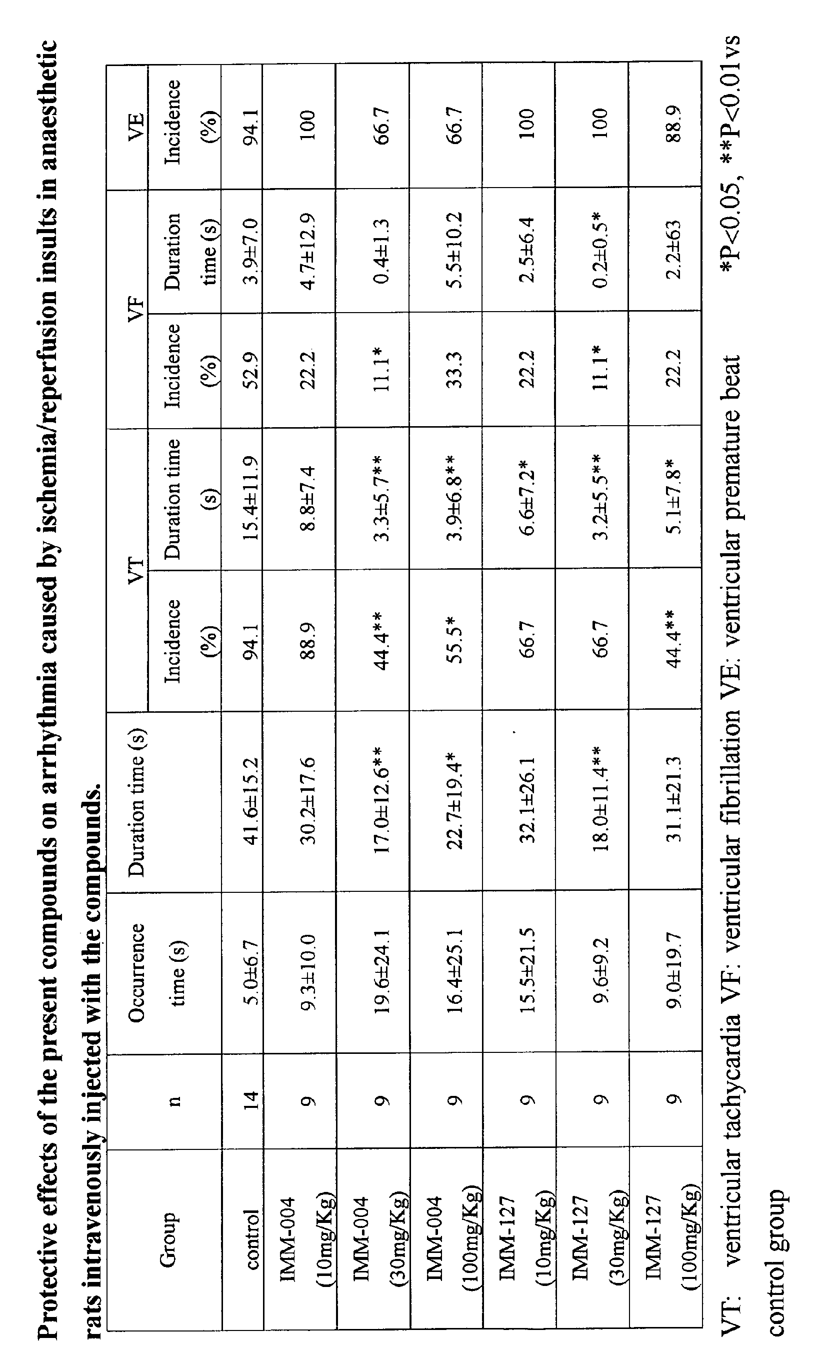

### (2) Protective effects of the tested compounds on myocardial insults in rats after orally administration

IMM-004 and IMM-127 were dissolved in the solution of ethanol and PEG (ethanol: PEG (v/v)= 1: 9). Rats were orally administrated the compounds at volume of 10ml/Kg. The left anterior descending coronary artery was ligated in rats for 15min at 60 min after the administration, and then the reprefusion was achieved.

### 1) IMM-004

No protective effect was observed in rats orally administrated IMM-004 at 10mg/Kg.

The protective effect of IMM-004 on the recovery of cardiovascular function was observed in rats after orally administration of 30mg/Kg body weight. Incidence of VT (P<0.01), VF (P<0.05), and duration of VT (P<0.01), as well as the duration of ventricular arrhythmic (P<0.01) were all reduced.

Incidence of VT (P<0.05) and duration of VT (P<0.01), as well as the duration of ventricular arrhythmic (P<0.05) were all reduced at a dose of 100mg/kg.

### 2) IMM-127

Duration of VT (P<0.05) was significantly reduced at a dose of 10mg/Kg.

Incidence of VF (P<0.05) and duration of VT (P<0.01) and VF ((P<0.05), as well as the duration of ventricular arrhythmic (P<0.01) were all reduced at a dose of 30mg/kg.

Incidence of VT (P<0.05) and duration of VT (P<0.01) were reduced at a dose of 100mg/kg.

The protective effect of compounds mentioned above on myocardial damage caused by ischemia/reprefusion was observed in rats orally administrated at 10, 30 and 100 mg/kg body weight. Arrhythmic potency induced by myocardial insults was used to assess the potential protective effect of the compounds.

Conclusion: On the basis of above experiments, the protective effect on myocardial ischemic injury was observed in rats intravenously and orally administrated the tested compounds.

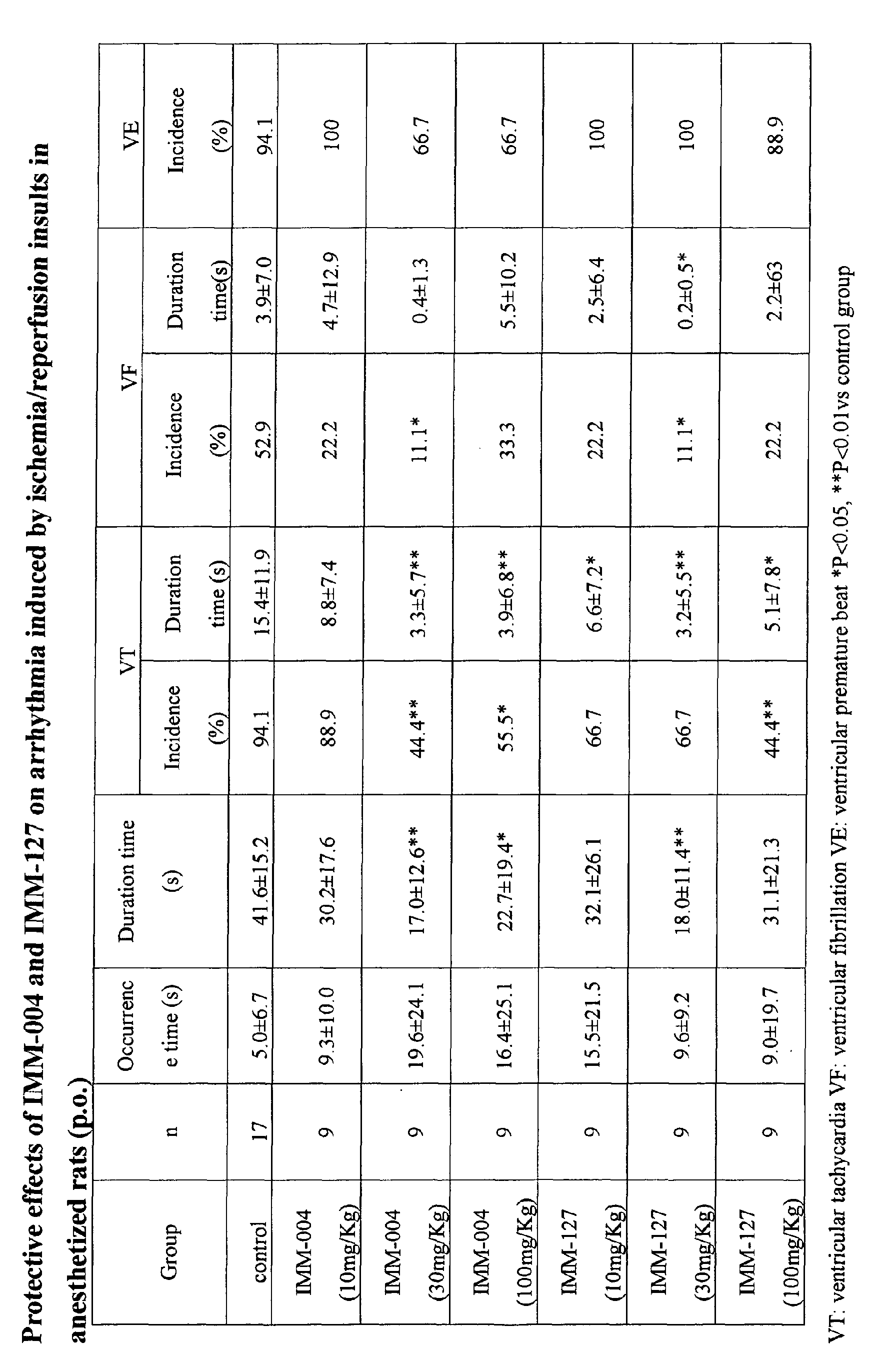

### Example 3: Protective effects of the compounds on myocardial ischemia in anesthetized dogs

Effects of IMM-04 on acute myocardial ischemia, infarction were observed in dogs. Dogs were treated with IMM-004 via duodenum, and parameters including the severity (Σ-ST) and area (NST) of myocardial ishemia revealed by epicardial electrography, myocardial infarct size represented by N-BT staining method, Coronary flow, oxygen consumption, the activity of CK, LDH, AST in blood, and change of plasm ET, TXB₂ and 6-Keto-PGF₁ were observed.

Dogs were divided into three groups: 1, vehicle control group, PEG 1ml/kg, n=5; 2, dilthiazem group, 5mg/kg, n=5; 3, IMM-004 group, 30mg/kg, n=5. The tested drugs were prepared in distilled water at volume of 1ml/kg. The tested items were administrated via duodenum by animals.

### (1) Effects of compounds on myocardial infarction area and ischemic injury reduction

### 1) Effects of compounds on severity of myocardial ischemia (Σ-ST)

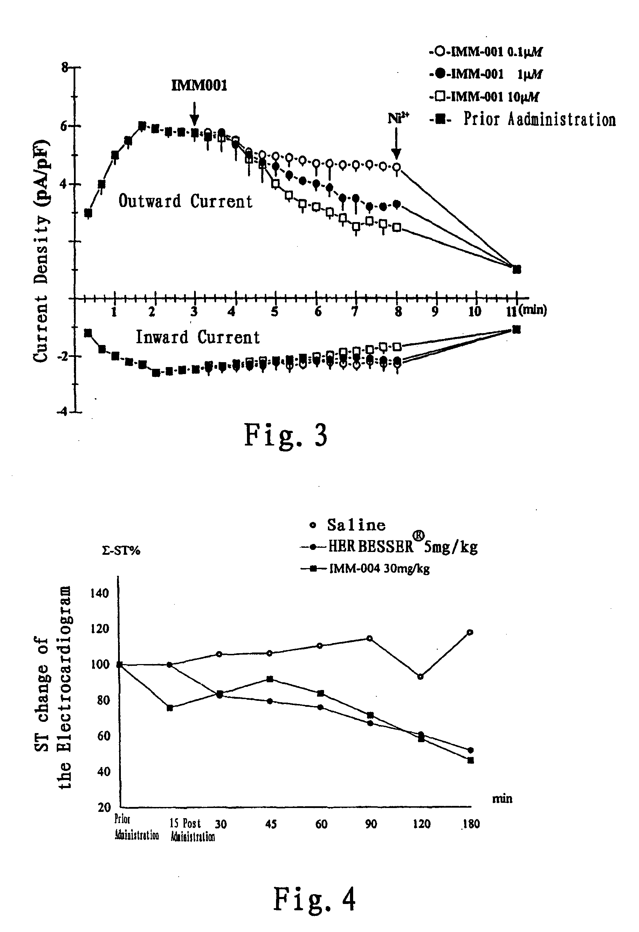

No modification of severity of myocardial ischemia (Σ-ST) was observed in vehicle (PEG) control group. Σ-ST in dilthiazem (blocker of calcium channel, commercially available as HERBESSER®) group was reduced 90 min after the administration and lasted for 180 min after the administration. Significant reduction of myocardial ischemia (Σ-ST) was observed in animals treated with IMM-004 via duodenum at a dose of 30mg/kg. Σ-ST decreased from 325.20±55.95mV to 146.80±60.25mV 180min after administration of IMM-004. Σ-ST decreased by 53.90± 20.60%. There were significant difference among compound-treated and vehicle control group (P<0.01). See Fig. 4.

The results obtained from epicardial electrography (EEG) provided the evidence of the protective effect of IMM-004 on experimental acute myocardial ischemia in dog. Severity (Σ-ST) and area (N-ST) of myocardial ischemia obtained from epicardial electrography (EEG) detection and myocardial infarction size represented by N-BT staining method were all reduced by compound treatment.

### 2) Effects on myocardial infarction area in dogs (N-BT method)

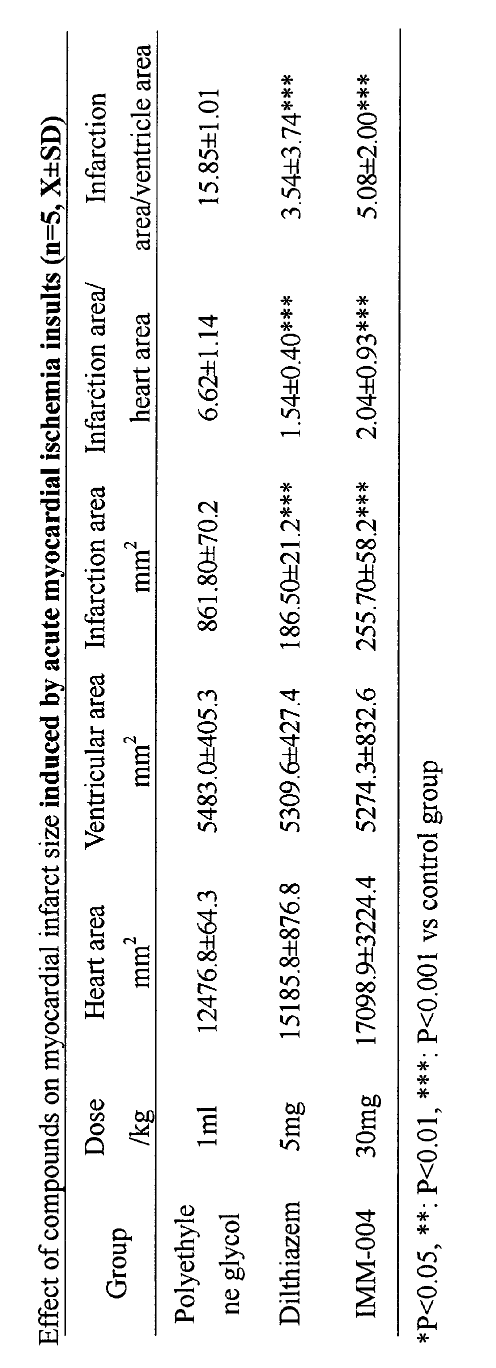

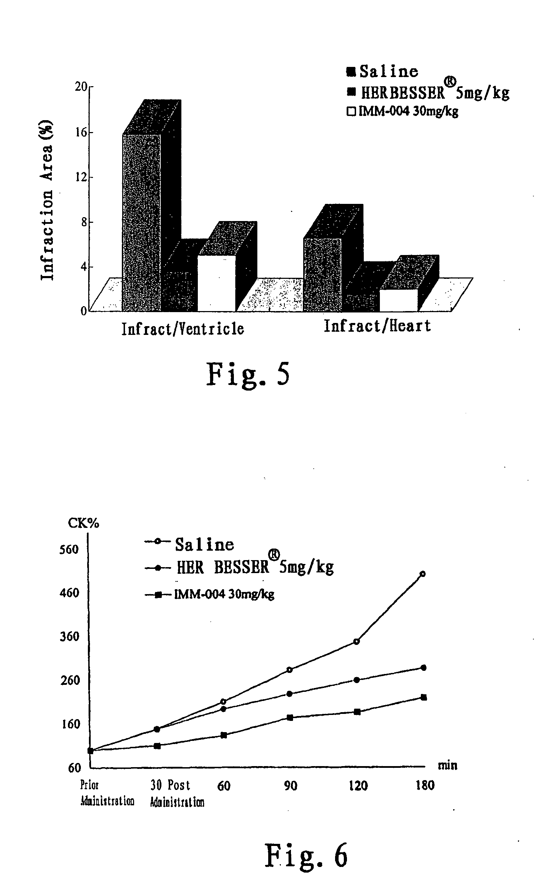

Myocardial infarct size represented by quantitive N-BT staining method, Infarction size was 6.62±1.14% of whole heart and 15.85±1.01% of ventricle in vehicle control group. Myocardial infarction size was significantly reduced by IMM-004 and dilthiazem, the positive control. Myocardial infarction size was reduced to 2.04±0.93% of heart and 5.08±2.00% of ventricular by IMM-004 treatment at a dose of 30mg/kg, which is decreased to 69.18% and 67.94% compared with the vehicle control. The significant difference was observed between two groups (P<0.001). See Fig 5.

### 3) Effects on the change of blood biochemical parameters (serum CK and LDH) in dog subjected to myocardial ischemia insult

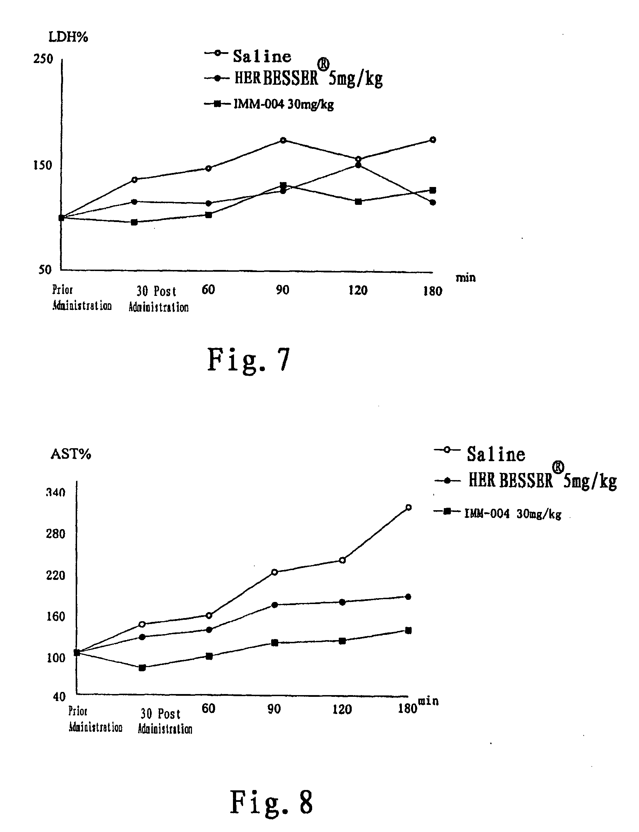

Serum CK, LDH and AST concentrations were significantly elevated in dogs subjected to acutely myocardial ischemia insult. IMM-004 treatment significantly inhibited the elevation of CK and AST concentrations, but showed no effect on the elevation of LDH activity compared with vehicle control group. The results are shown in Fig6, 7 and 8.

The results provided the evidence of the protective effect of IMM-004 on experimental acute myocardial ischemia and infarction in dogs. Severity (Σ-ST) and area (N-ST) of myocardial ischemia obtained from epicardial electrography (EEG) detection and myocardial infarction size represented by N-BT staining method were all reduced.

Creatine kinase (CK) is widely distributed in cytoplasm, especially cytoplasm of cardiomyocytes. CK in cardiomyocytes was released into blood after cardiovascular injury, such results in the elevation of serum CK activity. Therefore, the release of cytosolic CK from the cardiocytes is a reliable indicator of ischemic cell injury. Cytosolic LDH was released by damaged cardiocytes into the fluid after myocardial infarction. The elevation of coronary venous sinus blood LDH concentration represents the severity of myocardial injury. The current study demonstrated that IMM-004 treatment significantly inhibited the elevation of CK activity induced by cardiomyocytes damage. The experiment also demonstrated that cytosolic AST was released by damaged cardiocytes after myocardial injury, and IMM-004 treatment significantly inhibited the elevation of blood AST concentration.

### (2) Dilating Effects on coronary artery

### 1) Effect of compound on coronary blood flow in dog subjected to experimental acute myocardial ischemia

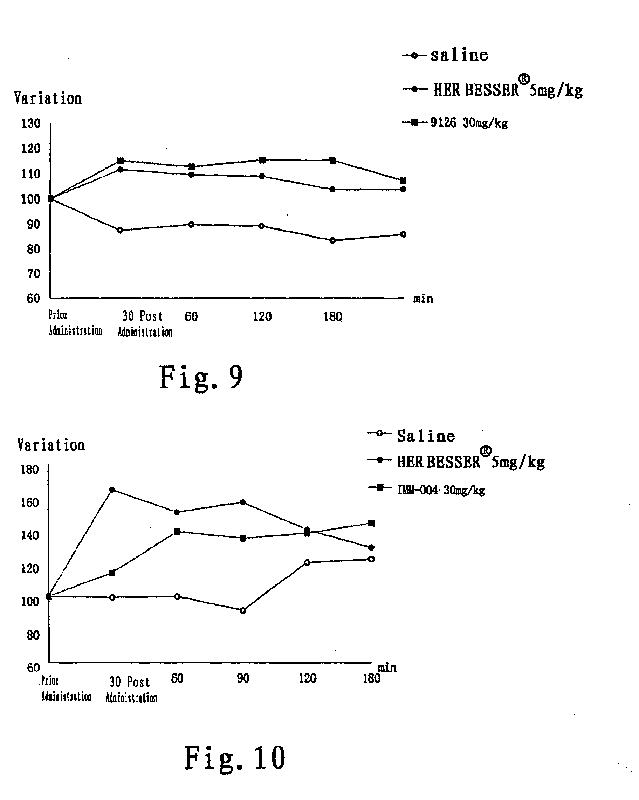

Coronary blood flow was transiently and compensatively increased by 10% after acute myocardial ischemia in dogs subjected to ligative exercise. Coronary flows were significantly increased after dilthiazem or IMM-004 treatment. Coronary flow was significantly increased by 15.02±22.16% verse the baseline 30 min after IMM-004 treatment, with significant difference to control group (P<0.05). See Fig9.

### 2) Effects on arterial and venous blood oxygen content in dogs subjected to experimental myocardial ischemia

No change of arterial and venous blood oxygen content and myocardial oxygen consumption was observed in vehicle (PEG) control group before and after administration. Significant increase of blood oxygen concentration and decrease of myocardial oxygen consumption were observed in dilthiazem-treated group. Coronary flow was significantly increased in IMM-004 treated group at 120min and 180min after the administration, with significant difference to the baseline obtained before administration (P<0.05). The results are shown in Fig10.

Coronary artery stenosis and occlusion induced by diverse pathophysiological insults can result in myocardial ischemia and infarction. In such a state, collateral circulations are compensatively developed in order to reduce the severity of myocardial ischemia and infarction. IMM-004 treatment increased coronary blood flow in dogs subjected to myocardial ischemia and infarction. Vasodilating effect of the compound on coronary artery, promoting effect of the compound on collateral circulation and blood supply on dogs subjected to ischemic myocardium were also observed in drug-treated group.

The above data shows that, pathological manifestation of acute myocardial ischemia and infarction are inhibited by IMM-004 treatment in dogs. Severity of myocardial ischemia and myocardial infarction size are reduced. In addition, dilating effect of the compound on coronary artery was observed, as well as coronary blood flow was increased by IMM-004 treatment at the mean time. The compound also demonstrates inhibitory effects on the release of CK and AST induced by ischemia injury. The above results are coincided with the pharmacological mechanism of its action, that the drug is a selective blocker of reverse mode Na⁺/Ca²⁺ exchange process. The pharmacological actions including protective effect on ischemia injury, dilating effect on coronary artery, and blood supply increasing, were all in accord with its mechanism of action.

### Example 4: Improvement of cardiac function and homodynamic in rats

### (1) Effects on cardiac function and homodynamic in normal rats

Method: Male SD rats, weighing 300-350g were fasted for 12 hrs and anesthetized by i.p. treatment with 20% Urethane (0.5ml/100g, 1g/kg). Polyethylene tubes (PE-50) were inserted into the left femoral artery, left femoral vein, right femoral vein and the left ventricle. The left femoral artery was cannulated for blood collection, and the left femoral vein was cannulated for liquid compensation, the right femoral artery was cannulated for blood pressure detection, the left ventricle was cannulated for ventricular pressure detection, the left femoral artery and ventricle were cannulated and the catheter was linked with pressure transducer by triplet. Signals were acquired by oscillograph and multi-channel biodynamic recorder linked with the pressure transducer. Rats in drug-treating group were orally administered with IMM-004 at 30 mg/kg, while rats in vehicle control group was orally treated with equal volume of vehicle (PEG). Left ventricular systolic pressure (LVSP), left ventricular end-diastolic pressure (LVEDP), and maximum rate of left ventricular pressure (±dp/dtmax), artery systolic pressure (SP), artery diastolic pressure (DP), mean artery pressure (MAP) and heart rate (HR) were recorded 40 min after the administration. Signals were continuously recorded in every 10 min.

Results: Left ventricular systolic pressure (LVSP), left ventricular end-diastolic pressure (LVEDP), and maximum rate of left ventricular pressure (±dp/dtmax), artery systolic pressure (SP), artery diastolic pressure (DP), mean artery pressure (MAP) and heart rate (HR) were not significantly influenced by IMM-004, indicating that IMM-004 had no effect on cardiac function and hemodynamics in rats without treatment. There is no significant statistic difference between vehicle control group and IMM-004 treated group.

Conclusion: IMM-004 has no effect on cardiac function and hemodynamics.

### (2) Effects on the recovery of cardiac function and hemodynamics in rats subjected to myocardial ischemia/reperfusion insult

Method: Male SD rats weighing 300-350g were fasted for 12 hrs and anesthetized by i.p. treatment with 20% Urethane (0.5ml/100g, 1g/kg). Polyethylene tubes (PE-50) were inserted into the left femoral artery, left femoral vein, right femoral vein and the left ventricle. The left femoral artery was cannulated for blood collection, and the left femoral vein was cannulated for liquid compensation, the right femoral artery was cannulated for blood pressure detection, the left ventricle was cannulated for ventricular pressure detection, left femoral artery and ventricle were cannulated and the catheter was linked with pressure transducer by triplet. Signals were acquired by oscillograph and multi-channel biodynamic recorder linked with the pressure transducer. Rats in drug-treated group were orally treated with IMM-004 at 30 mg/kg, while rats in vehicle control group was orally treated with an equal volume of vehicle (PEG). Dissect the trachea and artificial respiration was performed via respirator after thoracotomy (2ml/100g). A left thoracotomy was performed from the forth to fifth intercostals space by cutting off the forth and fifth rib 2mm from the left border of sternum, and the pericardium was opened to expose the heart. A 3.0 silk ligature was placed under the left anterior descending coronary artery. The heart was taken back to the chest and allowed to stabilize for 10 min. The ends of the suture were threaded through a polyethylene tube (3x8mm) to form a snare. Pull the ends of the suture taut and clamp the snare with a hemostat to occlude the coronary artery. Coronary artery occlusion was verified by epicardial cyanosis and subsequent decrease in arterial blood pressure. Reprefusion of the heart was initiated via unclamping the hemostat and loosening the snare for 15 min. Left ventricular systolic pressure (LVSP), left ventricular end-diastolic pressure (LVEDP), and maximum rate of left ventricular pressure (±dp/dtmax), artery systolic pressure (SP), artery diastolic pressure (DP), mean artery pressure (MAP) and heart rate (HR) were continuously recorded every 10 min before and after thoracotomy and during the period of ischemia (15 min) and reperfusion (1h).

Results: LVSP was decreased steadily from 120mmHg to 100mmHg in the IMM-004 treated group. LVSP was decreased to 60.8mmHg with fluctuation in the vehicle control group. LVEDP was rapidly increased 10min after reperfusion in control group, indicating the diastolic dysfunction and relevant damage of left ventricle (+15mmHg). LVEDP was steadily equal to the baseline in IMM-004 group (about -6mmHg), demonstrating the potent protective effect of IMM-004 on myocardial diastolic function. +dp/dtmax is a parameter usually used to assess myocardial contractible function. +dp/dtmax in IMM-004 group was about +110mmHg/s, which was decreased without significance. +dp/dtmax in the vehicle control group decreased to 50mmHg/s, is 54% lower than that of the IMM-004 group. -dp/dtmax, is a parameter used to assess myocaridial diastolic function. -dp/dtmax in the IMM-004 group reduced from 100mmHg/s to 70mmHg/s, and -dp/dtmax in the vehicle group reduced from 100mmHg/s to 30mmHg/s. The results indicated that IMM-004 has significantly protective effect on myocardial contractible dysfunction, and myocardial diastolic function was preserved to normal level.

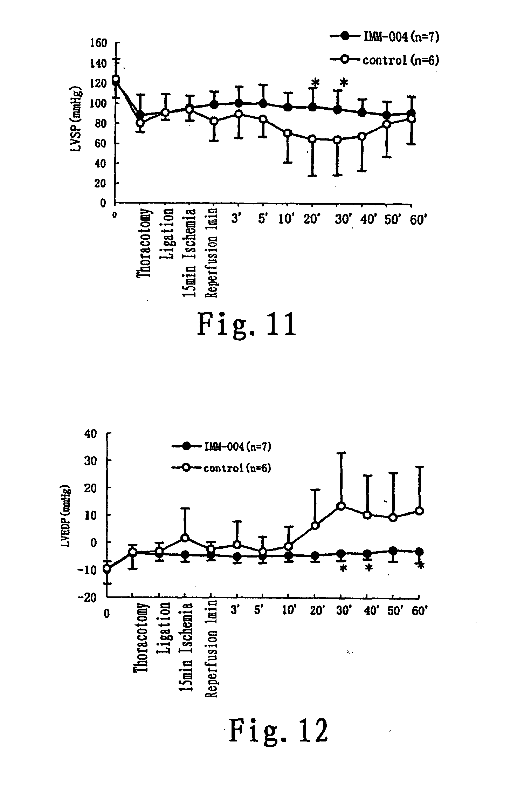

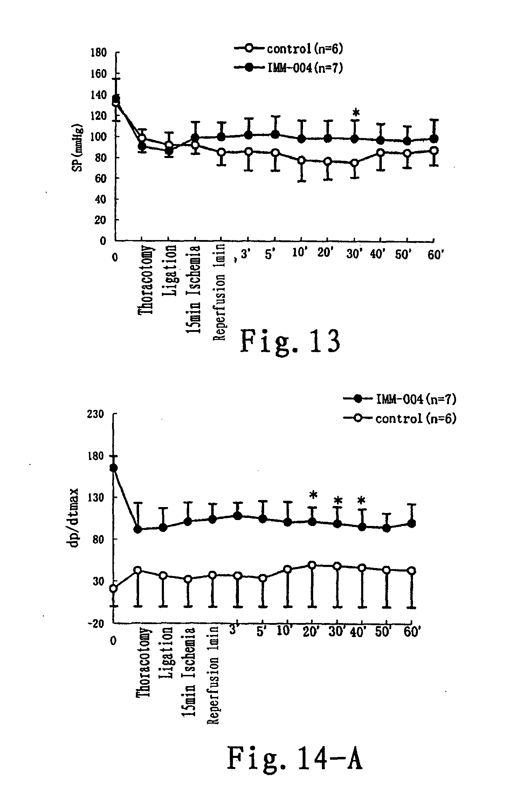

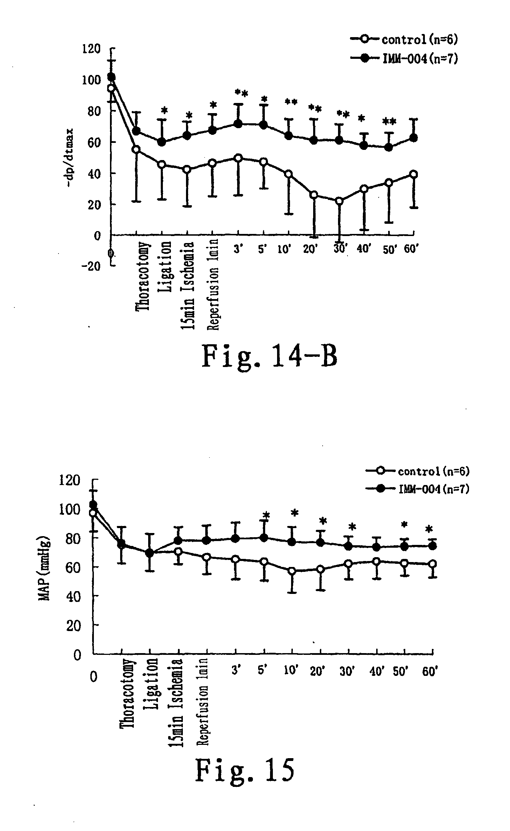

IMM-004 shows significant protective effect on cardiac function, thus blood pressure of rats in the IMM-004 group was stable (SBP 100mmHg, MAP 80mmHg). Compared with the IMM-004 group, blood pressure of rats in the vehicle control group reduced and fluctuated in a large scale (SBP 80mmHg, MAP60 mmHg), with sever myocardial damage developed in rats subjected to ischemia/reprefusion. As shown in figure, heart rate of rats subjected to ischemia/reprefusion decreased (from 461 to 400beats/min). Heart rate was not influenced by IMM-004 treatment and there was no difference between two groups. See Figs 11, 12, 13, 14 and 15.

Conclusion: Cardiac function of rats subjected to ischemia/reprefusion insult is reduced, represented by the decrease of myocardial diastolic function and blood pressure, or blood pressure is fluctuating in a large-scale. IMM-004 shows significant protective effect on cardiac dysfunction induced by ischemia/reprefusion, with more potent effect on the recovery of cardiac diastolic function to normal level. Blood pressure of rats in IMM-004 group is stable. In addition, IMM-004 has no effect on heart rate decrease induced by myocardial injury.

### Example 5: Toxicity study

### (1) Ames (mutagenesis) Tests

To assess the mutagenic effects of 5 compounds (IMM-004;IMM-065; IMM-127; IMM-128; IMM-313), the Ames test was performed in 4 strains (TA97; TA98; TA100; TA102). The experiment was conducted with compounds at 4 concentration levels (0.5, 5, 50 and 500µg/dish). No evidence for a mutagenic effect of these compounds was found. Results are shown as follows.

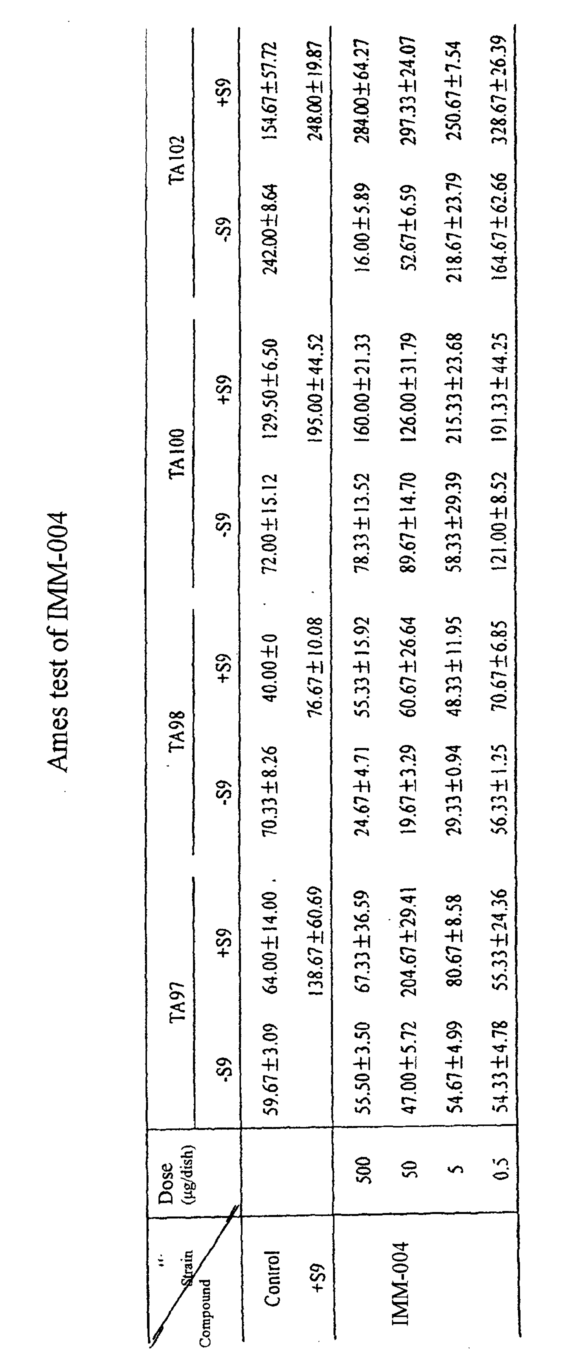

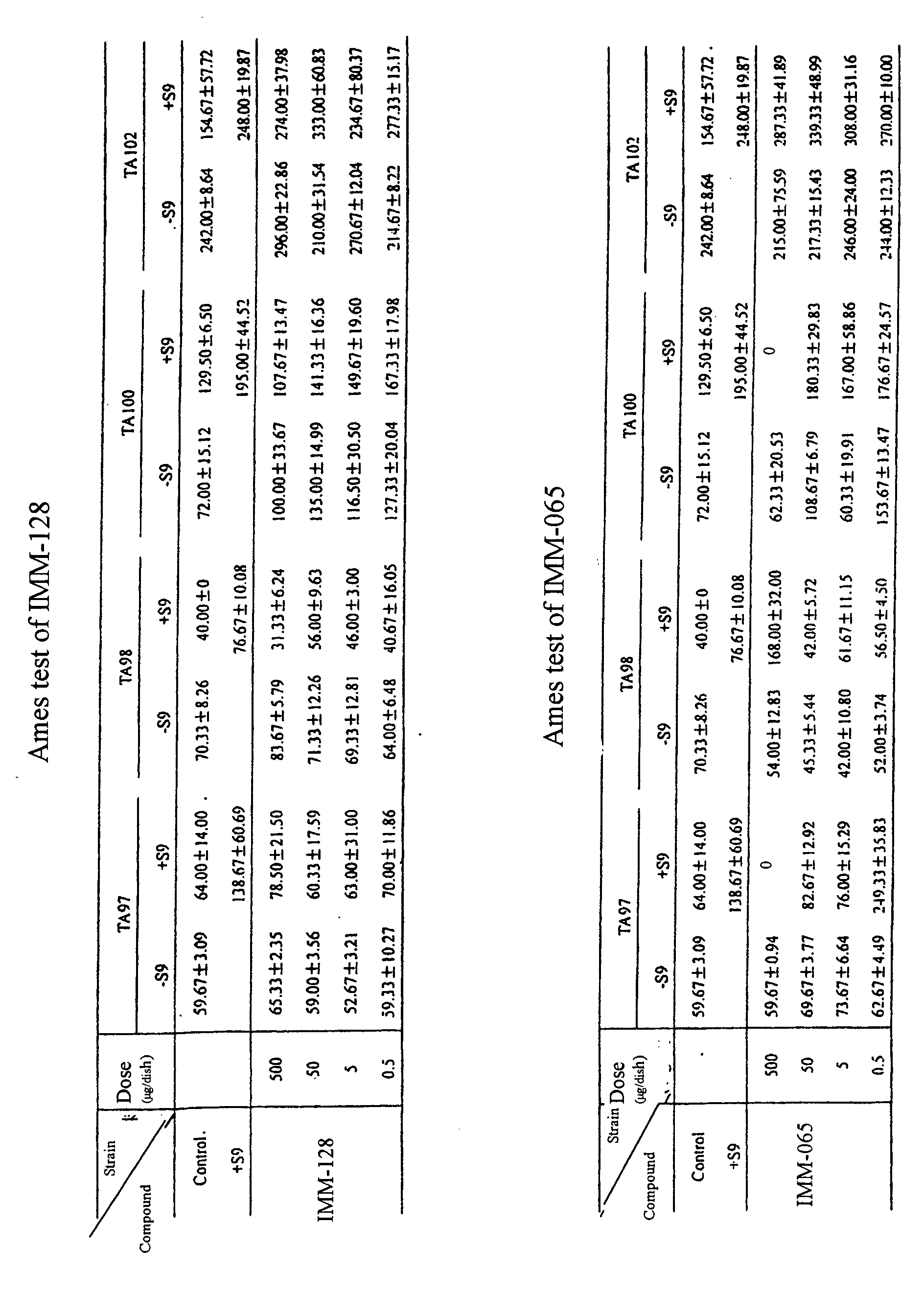

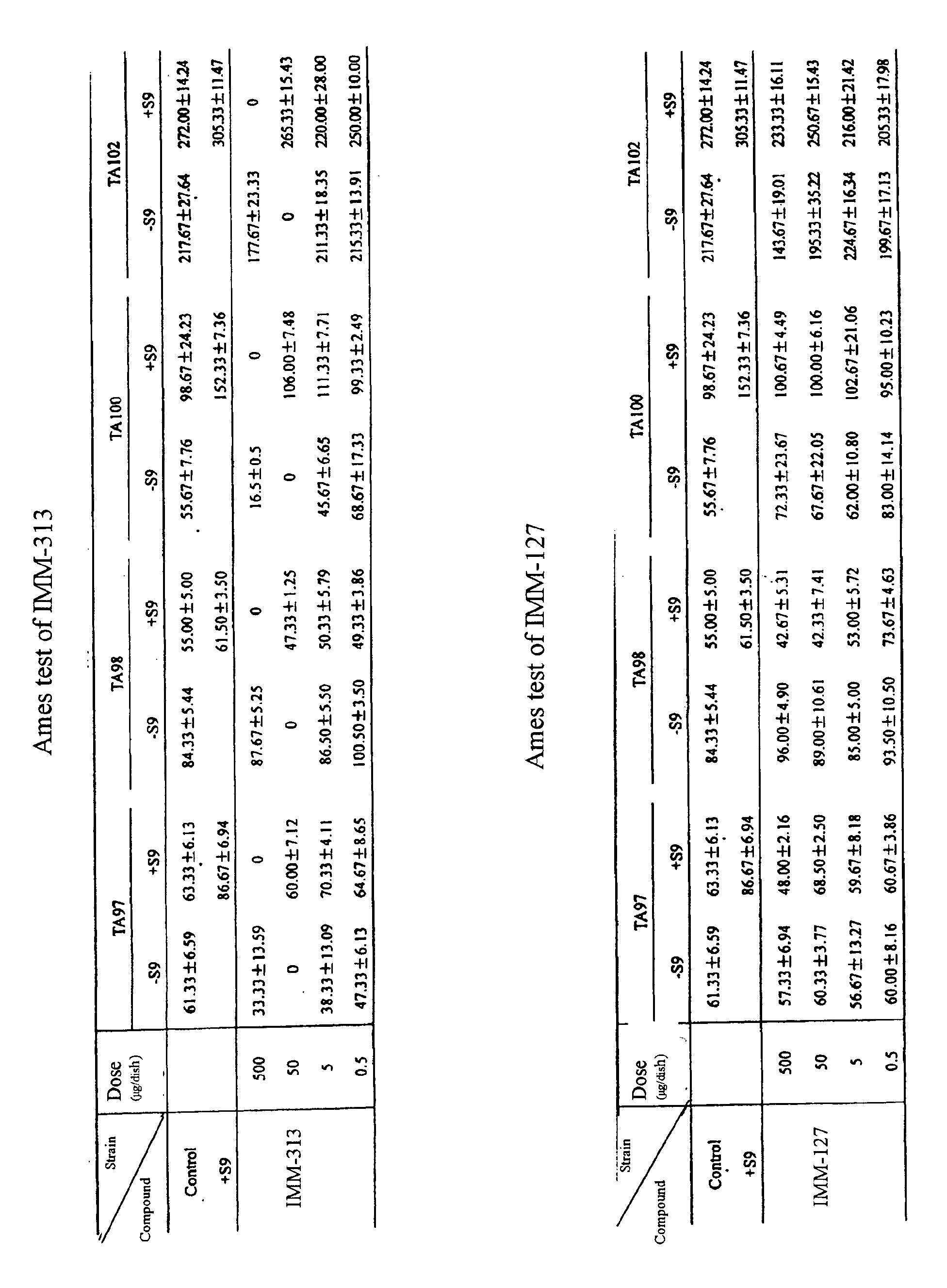

### (2) Acute toxicity study

Acute toxicity studies were performed with IMM-004 and IMM-127 (synthesized by ourselves). The compound was provided as powder, which was milled and suspended in 0.5% carboxymethylcellulose sodium (CMC-Na) before use.

Male KM mice weighting 19±2g were housed for an adaptation before use. Mice were randomly divided into groups, and each group consists of 10 animals. Mice were all fasted for 14 hrs before the administration of the tested compounds. Activity of mice in high dose group was reduced 30 min after the administration. 24h after the administration, mice in each group were restored to normal. Mice were observed daily for a period of 7 days after the administration. Results are shown as follows:

| Concentration | Lethal cases | |
|---|---|---|
| | **IMM-004** | **IMM-127** |
| 200mg/kg | -- | -- |
| 350mg/kg | -- | -- |
| 500mg/kg | -- | -- |
| 700mg/kg | -- | -- |
| 1g/kg | -- | -- |
| 2g/kg | -- | -- |
| 3g/kg | -- | |
| 5g/kg | -- | 7 |

Conclusion: There was no mortality in any of the dose groups of IMM-004, oral LD50 (50% lethal death) > 5g/kg, indicating the low toxicity of these compounds.

## Claims

1. Use of benzisoselenazolones in the preparation of medicament against ischemic myocardial injury.

2. Use as set forth in claim 1, **characterized** that said medicament targets Na⁺/Ca²⁺ exchanger.

3. Use as set forth in claim 1, **characterized** that said benzisoselenazolone is represented by formula (I), 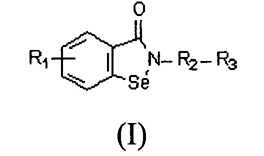 wherein R₁ is selected from a hydrogen, a halogen, a C₁-C₄ linear or branched alkyl, a C₁-C₄ linear or branched alkoxy, a hydroxy, a trifluoromethyl, a nitro, a di-(C₁-C₄ alkyl)-amino, or a methylenedioxy group, the substitution site of R₁ may be any one of the 3-, 4-, 5- or 6-position of the benzene ring of benzisoselenolone;
R₂ is a C₀-C₄ saturated or unsaturated alkyl, where R₂ is a bond connecting R3 and nitrogen when R₂ being C₀;
R3 represents a saturated or unsaturated, substituted or unsubstituted phenyl or heterocyclic radical, said heterocyclic radical has 1 to 4 hetero atoms being selected from oxygen, nitrogen or sulphur.

4. Use as set forth in claim 3, **characterized** that said benzisoselenazolone is represented by formula (I), 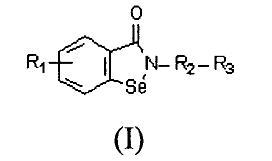 wherein R₁ is selected from a hydrogen, a halogen, a C₁-C₄ linear or branched alkyl, a C₁-C₄ linear or branched alkoxy, a hydroxy, a trifluoromethyl, a nitro, a di-(C₁-C₄ alkyl)-amino, or a methylenedioxy group, the substitution site of R₁ may be any one of the 3-, 4-, 5- or 6-position of the benzene ring of benzisoselenolone;
R₂ is a C₀-C₂ saturated or unsaturated alkyl, where R₂ is a bond connecting R3 and nitrogen while R₂ being C₀;
R3 represents a saturated or unsaturated, substituted or unsubstituted phenyl or heterocyclic radical, said heterocyclic radical has 1 to 2 hetero atoms selected from oxygen, nitrogen or sulphur and said heterocyclic radical may be mono- or di- substituted where the substituents may be identical or different, being selected from the group consisting of halogen, C₁-C₄ linear or branched alkyl, C₁-C₄ linear or branched alkoxy, C₁-C₄ linear or branched alkylthio, C₁-C₄ linear or branched keto, C₁-C₄ linear or branched haloalkyl, hydroxy, thio, nitro, cyano, carboxyl, and alkoxycarbonyl.

5. Use as set forth in claim 4, **characterized** that said benzisoselenazolone is represented by formula (I), 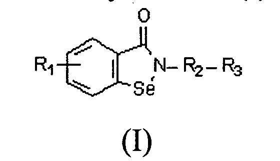 wherein R₁ is selected from a hydrogen, a halogen, a C₁-C₄ linear or branched alkyl, a C₁-C₄ linear or branched alkoxy, a hydroxy, a trifluoromethyl, a nitro, a di-(C₁-C₄ alkyl)-amino, or a methylenedioxy group, the substitution site of R₁ may be any one of the 3-, 4-, 5- or 6-position of the benzene ring of benzisoselenolone;
R₂ is a bond connecting R3 and nitrogen;
R3 represents a substituted or unsubstituted, saturated or unsaturated phenyl or heterocyclic radical, said heterocyclic having 1 to 2 hetero atoms selected from oxygen, nitrogen or sulphur, and may be mono- or disubstituted, where the substituent being identical or different, being selected from the group consisting of halogen, C₁-C₄ linear or branched alkyl, C₁-C₄ linear or branched alkoxy, C₁-C₄ linear or branched alkylthio, C₁-C₄ linear or branched haloalkyl, C₁-C₄ linear or branched keto, hydroxy, thio, nitro, cyano, carboxyl, and alkoxycarbonyl.

6. Use as set forth in claim 5, **characterized** that said benzisoselenazolone is represented by formula (I), 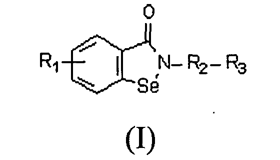 wherein R₁ is selected from a hydrogen, a halogen, a C₁-C₄ linear or branched alkyl, a C₁-C₄ linear or branched alkoxy, a hydroxy, a trifluoromethyl, a nitro, a di-(C₁-C₄ alkyl)-amino, or a methylenedioxy group, the substitution site of R₁ may be any one of the 3-, 4-, 5- or 6-position of the benzene ring of benzisoselenolone;
R₂ is a bond connecting R3 and nitrogen;
R3 represents a substituted or unsubstituted phenyl, phenylthio, thiazolyl, isothiazolyl, imidazolyl, pyrazolyl, thiadiazolyl, pyridyl, pyrimidyl, pyrazinyl, pyridazinyl, benzothiazolyl, benzimidazolyl, benzotriazolyl, triazinyl, triazolyl, tetrazolyl, quinolinyl, isoquinolinyl, indolyl, indazolyl, carbazolyl, furyl, which may be mono- or di- substituted, where the substituent being identical or different, and is selected from the group consisting of halogen, C₁-C₄ linear or branched alkyl, C₁-C₄ linear or branched alkoxy, C₁-C₄ linear or branched alkylthio, C₁-C₄ linear or branched haloalkyl, C₁-C₄ linear or branched keto, hydroxy, thio, nitro, cyano, carboxyl, and alkoxycarbonyl.

7. Use as set forth in claim 6, **characterized** that said benzisoselenazolone is represented by formula (I), 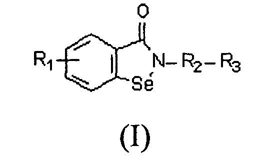 wherein R₁ is selected from a hydrogen, a halogen, a C₁-C₄ linear or branched alkyl, a C₁-C₄ linear or branched alkoxy, a hydroxy, a trifluoromethyl, a nitro, a di-(C₁-C₄ alkyl)-amino, or a methylenedioxy group, the substitution site of R₁ may be any one of the 3-, 4-, 5- or 6-position of the benzene ring of benzisoselenolone;
R₂ is a bond connecting R3 and nitrogen;
R3 represents a substituted or unsubstituted phenyl or pyridyl, which may be mono- or di- substituted, where the substituent being identical or different, and is selected from fluoro, chloro, bromo, methyl, methoxy, ethoxy, methylthio, hydroxy, thio, nitro, carboxyl, methoxycarbonyl and ethoxycarbonyl.

8. Use as set forth in claim 1, **characterized** that said benzisoselenazolone is selected from:
2-phenyl-1, 2-benzisoselenazol-3(2H)-one,
4-fluoro-2-phenyl-1, 2-benzisoselenazol-3(2H)-one,
5-fluoro-2-phenyl-1, 2-benzisoselenazol-3(2H)-one,
6-fluoro-2-phenyl-1, 2-benzisoselenazol-3(2H)-one,
7-fluoro-2-phenyl-1, 2-benzisoselenazol-3(2H)-one,
4-chloro-2-phenyl-1 2-benzisoselenazol-3(2H)-one,
5-chloro-2-phenyl-1, 2-benzisoselenazol-3(2H)-one,
6-chloro-2-phenyl-1, 2-benzisoselenazol-3(2H)-one,
7-chloro-2-phenyl-1, 2-benzisoselenazol-3(2H)-one,
4-hydroxy-2-phenyl-1, 2-benzisoselenazol-3(2H)-one,
5-hydroxy-2-phenyl-1, 2-benzisoselenazol-3(2H)-one,
6-hydroxy-2-phenyl-1, 2-benzisoselenazol-3(2H)-one,
7-hydroxy-2-phenyl-1, 2-benzisoselenazol-3(2H)-one,
2-(2-pyridyl)-1, 2-benzisoselenazol-3(2H)-one,
2-(3-pyridyl)-1, 2-benzisoselenazol-3(2H)-one,
2-(4-pyridyl)-1, 2-benzisoselenazol-3(2H)-one,
4-chloro-2-(2-pyridyl)-1, 2-benzisoselenazol-3(2H)-one,
4-chloro-2-(3-pyridyl)-1, 2-benzisoselenazol-3(2H)-one,
4-chloro-2-(4-pyridyl)-1, 2-benzisoselenazol-3(2H)-one,
5-chloro-2-(2-pyridyl)-1, 2-benzisoselenazol-3(2H)-one,
5-chloro-2-(3-pyridyl)-1, 2-benzisoselenazol-3(2H)-one,
5-chloro-2-(4-pyridyl)-1, 2-benzisoselenazol-3(2H)-one,
6-chloro-2-(2-pyridyl)-1, 2-benzisoselenazol-3(2H)-one,
6-chloro-2-(3-pyridyl)-1, 2-benzisoselenazol-3(2H)-one,
6-chloro-2-(4-pyridyl)-1, 2-benzisoselenazol-3(2H)-one,
7-chloro-2-(2-pyridyl)-1, 2-benzisoselenazol-3(2H)-one,
7-chloro-2-(3-pyridyl)-1, 2-benzisoselenazol-3(2H)-one,
7-chloro-2-(4-pyridyl)-1, 2-benzisoselenazol-3(2H)-one,
4-fluoro-2-(2-pyridyl)-1, 2-benzisoselenazol-3(2H)-one,
4-fluoro-2-(3-pyridyl)-1, 2-benzisoselenazol-3(2H)-one,
4-fluoro-2-(4-pyridyl)-1, 2-benzisoselenazol-3(2H)-one,
5-fluoro-2-(2-pyridyl)-1, 2-benzisoselenazol-3(2H)-one,
5-fluoro-2-(3-pyridyl)-1, 2-benzisoselenazol-3(2H)-one,
5-fluoro-2-(4-pyridyl)-1, 2-benzisoselenazol-3(2H)-one,
6-fluoro-2-(2-pyridyl)-1, 2-benzisoselenazol-3(2H)-one,
6-fluoro-2-(3-pyridyl)-1, 2-benzisoselenazol-3(2H)-one
6-fluoro-2-(4-pyridyl)-1, 2-benzisoselenazol-3(2H)-one,
7-fluoro-2-(2-pyridyl)-1, 2-benzisoselenazol-3(2H)-one,
7-fluoro-2-(3-pyridyl)-1, 2-benzisoselenazol-3(2H)-one,
7-fluoro-2-(4-pyridyl)-1, 2-benzisoselenazol-3(2H)-one,
4-hydroxy-2-(2-pyridyl)-1, 2-benzisoselenazol-3(2H)-one,
4-hydroxy-2-(3-pyridyl)-1, 2-benzisoselenazol-3(2H)-one,
4-hydroxy-2-(4-pyridyl)-1, 2-benzisoselenazol-3(2H)-one,
5-hydroxy-2-(2-pyridyl)-1, 2-benzisoselenazol-3(2H)-one,
5-hydroxy-2-(3-pyridyl)-1, 2-benzisoselenazol-3(2H)-one,
5-hydroxy-2-(4-pyridyl)-1, 2-benzisoselenazol-3(2H)-one,
6-hydroxy-2-(2-pyridyl)-1, 2-benzisoselenazol-3(2H)-one,
6-hydroxy-2-(3-pyridyl)-1, 2-benzisoselenazol-3(2H)-one,
6-hydroxy-2-(4-pyridyl)-1, 2-benzisoselenazol-3(2H)-one,
7-hydroxy-2-(2-pyridyl)-1, 2-benzisoselenazol-3(2H)-one,
7-hydroxy-2-(3-pyridyl)-1, 2-benzisoselenazol-3(2H)-one,
7-hydroxy-2-(4-pyridyl)-1, 2-benzisoselenazol-3(2H)-one,
2-(3-hydroxy-2-pyridyl)-1, 2-benzisoselenazol-3(2H)-one,
2-(2-chloro-3-pyridyl)-1, 2-benzisoselenazol-3(2H)-one,
2-(5-chloro-2-pyridyl)-1, 2-benzisoselenazol-3(2H)-one,
2-(6-methoxy-3-pyridyl)-1, 2-benzisoselenazol-3(2H)-one,
2-(2, 6-dichloro-3-pyridyl)-1, 2-benzisoselenazol-3(2H)-one,
2-(4, 6-dimethyl-2-pyridyl)-1, 2-benzisoselenazol-3(2H)-one,
2-(6-hydroxy-2-pyridyl)-1, 2-benzisoselenazol-3(2H)-one,
2-(3-nitro-2-pyridyl)-1, 2-benzisoselenazol-3(2H)-one,
2-(5-nitro-2-pyridyl)-1, 2-benzisoselenazol-3(2H)-one,
6-methyl-2-(2-pyridyl)-1, 2-benzisoselenazol-3(2H)-one,
6-methoxy-2-(2-pyridyl)-1, 2-benzisoselenazol-3(2H)-one,
5-nitro-2-(2-pyridyl)-1, 2-benzisoselenazol-3(2H)-one,
7-methoxy-2-(2-pyridyl)-1, 2--benzisoselenazol-3(2H)- one,
6, 7-methylenedioxy-2-(2-pyridyl)-1, 2-benzisoselenazol-3(2H)-one,
2-(3-methyl-2-pyridyl)-1, 2-benzisoselenazol-3(2H)-one,
2-(4-methyl-2-pyridyl)-1, 2-benzisoselenazol-3(2H)-one,
2-(5-methyl-2-pyridyl)-1, 2-benzisoselenazol-3(2H)-one,
2-(6-methyl-2-pyridyl)-1, 2-benzisoselenazol-3(2H)-one,
2-(3, 5-dichloro-2-pyridyl)-1, 2-benzisoselenazol-3(2H)-one,
2-(4-chloro-2-pyridyl)-1, 2-benzisoselenazol-3(2H)-one,
2-(4-carboxyl-5-chloro-2-pyridyl)-1, 2-benzisoselenazol-3(2H)-one,
2-(3-carboxyl-2-pyridyl)-1, 2-benzisoselenazol-3(2H)-one,
2-(2-tetrahydropyridyl)-1, 2-benzisoselenazol-3(2H)-one,
2-(2-pyrimidinyl)-1, 2-benzisoselenazol-3(2H)-one,
2-(2-thiazolyl)-1, 2-benzisoselenazol-3(2H)-one,
2-(benzothiazolyl)-1, 2-benzisoselenazol-3(2H)-one,
2-(4-methyl-2-pyrimidinyl)-1, 2-benzisoselenazol-3(2H)-one,
2-(5-nitro-2-pyrimidinyl)-1, 2-benzisoselenazol-3(2H)-one,
2-(4, 6-dimethyl-2-pyrimidinyl)-1, 2-benzisoselenazol-3(2H)-one,
(2, 6-dimethyl-4-pyrimidinyl)-1, 2-benzisoselenazol-3(2H)-one,
2-(5-ethoxy-2-ethylthio-4-pyrimidinyl)-1, 2-benzisoselenazol-3(2H)-one,
2-(5-ethoxycarbonyl-2-hydroxy-4-pyrimidinyl)-1,2-benzisoselenazol-3(2H)- one,
2-(5-carboxyl-4-pyrimidinyl)-1, 2-benzisoselenazol-3(2H)-one,
2-(6-chloro-2-methylthio-4-pyrimidinyl)-1, 2-benzisoselenazol-3(2H)-one,
2-(4-chloro-2-methylthio-6-pyrimidinyl)-1, 2-benzisoselenazol-3(2H)-one,
2-(4-chloro-6-methyl-2-pyrimidinyl)-1, 2-benzisoselenazol-3(2H)-one,
2-(6-chloro-3-nitro-2-pyrimidinyl)-1, 2-benzisoselenazol-3(2H)-one,
2-(2-chloro-5-nitro-4-pyrimidinyl)-1, 2-benzisoselenazol-3(2H)-one,
2-(4, 6-dichloro-2-pyrimidinyl)-1, 2-benzisoselenazol-3(2H)-one,
2-(4, 6-dichloro-5-pyrimidinyl)-1, 2-benzisoselenazol-3(2H)-one,
2-(4, 6-dihydroxy-2-pyrimidinyl)-1, 2-benzisoselenazol-3(2H)-one,
2-(2, 6-dihydroxy-4-pyrimidinyl)-1, 2-benzisoselenazol-3(2H)-one,
2-(2, 4-dihydroxy-5-pyrimidinyl)-1, 2-benzisoselenazol-3(2H)-one,
2-(4, 6-dithio-2-pyrimidinyl)-1, 2-benzisoselenazol-3(2H)-one,
2-(6-hydroxy-2-thio-4-pyrimidinyl)-1, 2-benzisoselenazol-3(2H)-one,
2-(6-hydroxy-2-methylmercapto-4-pyrimidinyl)-1, 2-benzisoselenazol-3 (2H)- one,
2-(4-hydroxy-6-methyl-2-pyrimidinyl)-1, 2-benzisoselenazol-3(2H)-one,
2-(2-hydroxy-5-methyl-4-pyrimidinyl)-1, 2-benzisoselenazol-3(2H)-one,
2-(1-benzopyrazolo(3, 4-d)pyrimidin-4-yl)-1, 2-benzisoselenazol-3(2H)-one,
2-(pyrazolo(3, 4-d)pyrimidin-4-yl)-1, 2-benzisoselenazol-3(2H)-one,
2-(6-hydroxypyrazolo(3, 4-d)pyrimidin-4-yl)-1, 2-benzisoselenazol-3(2H)- one,
2-(6-thiopyrazolo(3, 4-d)pyrimidin-4-yl)-1, 2-benzisoselenazol-3(2H)-one,
2-(2-hydroxy-4-pyrimidinyl)-1, 2-benzisoselenazol-3(2H)-one,
2-(4-chloro-2-benzothiazolyl)-1, 2-benzisoselenazol-3(2H)-one,
2-(4-methoxy-2-benzothiazolyl)-1, 2-benzisoselenazol-3(2H)-one,
2-(6-methoxy-2-benzothiazolyl)-1, 2-benzisoselenazol-3(2H)-one,
2-(4-methyl-2-benzothiazolyl)-1, 2-benzisoselenazol-3(2H)-one,
2-(2-methyl-5-benzothiazolyl)-1, 2-benzisoselenazol-3(2H)-one,
2-(6-nitro-2-benzothiazolyl)-1, 2-benzisoselenazol-3(2H)-one,
2-(6-bromo-2-benzothiazolyl)-1, 2-benzisoselenazol-3(2H)-one,
2-(5, 6-dimethyl-2-benzothiazolyl)-1, 2-benzisoselenazol-3(2H)-one,
2-(6-ethoxy-2-benzothiazolyl)-1, 2-benzisoselenazol-3(2H)-one,
6-methyl-2-(2-thiazolyl)-1, 2-benzisoselenazol-3(2H)-one,
6-chloro-2-(2-thiazolyl)-1, 2-benzisoselenazol-3(2H)-one,
6-methoxy-2-(2-thiazolyl)-1, 2-benzisoselenazol-3(2H)-one,
5-nitro-2-(2-thiazolyl)-1, 2-benzisoselenazol-3(2H)-one,
5-chloro-2-(2-thiazolyl)-1, 2-benzisoselenazol-3(2H)-one,
7-methoxy-2-(2-thiazolyl)-1, 2-benzisoselenazol-3(2H)-one,
6, 7-methylenedioxy-2-(2-thiazolyl)-1, 2-benzisoselenazol-3(2H)-one,
2-(4-methyl-2-thiazolyl)-1, 2-benzisoselenazol-3(2H)-one,
2-(5-nitro-2-thiazolyl)-1, 2-benzisoselenazol-3(2H)-one,
2-(2-thiazolinyl)-1, 2-benzisoselenazol-3(2H)-one,
2-(5-chloro-2-thiazolyl)-1, 2-benzisoselenazol-3(2H)-one,
2-(3-methyl-5-isothiazolyl)-1, 2-benzisoselenazol-3(2H)-one,
2-(2-imidazolyl)-1, 2-benzisoselenazol-3(2H) one,
2-(5-ethoxycarbonyl-2-imidazolyl)-1, 2-benzisoselenazol-3(2H)-one,
2-(3-pyrazolyl)-1, 2-benzisoselenazol-3(2H)-one,
2-(4-cyano-5-pyrazolyl)-1, 2-benzisoselenazol-3(2H)-one,
2-(4-ethoxycarbonyl-3-pyrazolyl)-1, 2-benzisoselenazol-3(2H)-one,
2-(4-ethoxycarbonyl-2-phenyl-3-pyrazolyl)-1, 2-benzisoselenazol-3(2H)-one,
2-(4-cyano-3-pyrazolyl)-1, 2-benzisoselenazol-3(2H)-one,
2-(2-phenyl-3-pyrazolyl)-1, 2-benzisoselenazol-3(2H)-one,
2-(1-phenyl-5-pyrazolyl)-1, 2-benzisoselenazol-3(2H)-one,
2-(1-phenyl-5-pyrazolinon-3-yl)-1, 2-benzisoselenazol-3(2H)-one,
2-(5-hydroxy-3-pyrazolyl)-1, 2-benzisoselenazol-3(2H)-one,
2-(1, 3, 4-thiadiazolyl-2-yl)-1, 2-benzisoselenazol-3(2H)-one,
2-(5-thio-1, 3, 4-thiadiazolyl-2-yl)-1, 2-benzisoselenazol-3(2H)-one,
2-(5-methyl-1, 3, 4-thiadiazolyl-2-yl)-1, 2-benzisoselenazol-3(2H)-one,
2-(5-trifluoromethyl-1, 3, 4-thiadiazolyl-2-yl)-1, 2-benzisoselenazol-3(2H)-one,
2-(5-tert-butyl-1, 3, 4-thiadiazolyl-2-yl)-1, 2-benzisoselenazol-3(2H)-one,
2-(2-pyrazinyl)-1, 2-benzisoselenazol-3(2H)-one,
2-(2-carboxyl-3-pyrazinyl)-1, 2-benzisoselenazol-3(2H)-one,
2-(2-pyridazinyl)-1, 2-benzisoselenazol-3(2H)-one,
2-(2-benzimidazolyl)-1, 2-benzisoselenazol-3(2H)-one,
2-(5, 6-dimethyl-2-benzimidazolyl)-1, 2-benzisoselenazol-3(2H)-one,
2-(5-benzotriazolyl)-1, 2-benzisoselenazol-3(2H)-one,
2-(7-chloro-1, 2, 4-benzotriazinyl-3-yl)-1, 2-benzisoselenazol-3(2H)-one,
2-(1, 2, 4-benzotriazinyl-3-yl)-1, 2-benzisoselenazol-3(2H)-one,
2-(7-bromo-1, 2, 4-benzotriazinyl-3-yl)-1, 2-benzisoselenazol-3(2H)-one,
2-(7-fluoro-1, 2, 4-benzotriazinyl-3-yl)-1, 2-benzisoselenazol-3(2H)-one,
2-(7-nitro-1, 2, 4-benzotriazinyl-3-yl)-1, 2-benzisoselenazol-3(2H)-one,
2-(2-benzothienyl)-1, 2-benzisoselenazol-3(2H)-one,
2-(3-benzothienyl)-1, 2-benzisoselenazol-3(2H)-one,
2-(2-thienyl)-1, 2-benzisoselenazol-3(2H)-one,
2-(2, 1, 3-benzothiadiazolyl-4-yl)-1, 2-benzisoselenazol-3(2H)-one,
2-(1, 2, 4-triazinyl-4-yl)-1, 2-benzisoselenazol-3(2H)-one,
2-(2, 6-dithio-1, 3, 5-triazinyl-4-yl)-1, 2-benzisoselenazol-3(2H)-one,
2-(5, 6-dimethyl-1, 2, 4-triazinyl-3-yl)-1, 2-benzisoselenazol-3(2H)-one, 2-(5, 6-diphenyl-1, 2, 4-triazinyl-3-yl)-1, 2-benzisoselenazol-3(2H)-one,
2-(1, 2, 4-triazolyl-4-yl)-1, 2-benzisoselenazol-3(2H)-one,
2-(5-thio-1, 2, 4-triazolyl-3-yl)-1, 2-benzisoselenazol-3(2H)-one,
2-(5-tetrazolyl)-1, 2-benzisoselenazol-3(2H)-one,
2-(5-quinolinyl)-1, 2-benzisoselenazol-3(2H)-one,
2-(2-methyl-4-quinolinyl)-1, 2-benzisoselenazol-3(2H)-one,
2-(6-nitro-5-quinolinyl)-1, 2-benzisoselenazol-3(2H)-one,
2-(1, 2, 3, 4-tetrahydroquinolin-8-yl)-1, 2-benzisoselenazol-3(2H)-one,
2-(2-quinolinyl)-1, 2-benzisoselenazol-3(2H)-one,
2-(3-quinolinyl)-1, 2-benzisoselenazol-3(2H)-one,
2-(8-quinolinyl)-1, 2-benzisoselenazol-3(2H)-one,
2(6-methoxy-8-quinolinyl)-1, 2-benzisoselenazol-3(2H)-one,
2-(1-isoquinolinyl)-1, 2-benzisoselenazol-3(2H)-one,
2-(5-isoquinolinyl)-1, 2-benzisoselenazol-3(2H)-one,
2-(5 -indolyl)-1, 2-benzisoselenazol-3(2H)-one,
2-(5-isoindolyl)-1, 2-benzisoselenazol-3(2H)-one,
2-(5-indazolyl)-1, 2-benzisoselenazol-3(2H)-one,
2-(6-chloro-3-indazolyl)-1, 2-benzisoselenazol-3(2H)-one
2-(6-indazolyl)-1, 2-benzisoselenazol-3(2H)-one,
2-(7-indazolyl)-1, 2-benzisoselenazol-3(2H)-one,
2-(9-ethyl-3-carbazolyl)-1, 2-benzisoselenazol-3(2H)-one,
2-[(3,4-dihydroimidazol-2-yl)-2-ethenyl]-1,2-benzisoselenazol-3(2H)-one,
2-(2-pyridylmethyl)-1, 2-benzisoselenazol-3(2H)-one,
2-(2-furylmethyl)-1, 2-benzisoselenazol-3(2H)-one,
2-[4-(2-hydroxyimino-ethyl)]-1, 2-benzisoselenazol-3(2H)-one, and
2-[4-(7-n-butyl-benzo(1, 2-c-dihydrofuran-2-one))]-1,
2-benzisoselenazol-3 (2H)-one.

9. Use of a composition comprising a benzisoselenazolone compound for the preparation of a medicament against ischemic myocardial injury.
